# EUROPEAN PATENT SPECIFICATION

(11) **EP 2 165 185 B1**
(45) Date of publication and mention of the grant of the patent: **19.08.2015**
(21) Application number: 08771583.5
(22) Date of filing: 20.06.2008
(51) Int. Cl.: G01N 21/64, G01N 33/58

(54) **CONTROLLED MODIFICATION OF SEMICONDUCTOR NANOCRYSTALS**
KONTROLLIERTE MODIFIKATION VON HALBLEITER-NANOKRISTALLEN
MODIFICATION CONTRÔLÉE DE NANOCRISTAUX DE SEMI-CONDUCTEUR

(30) Priority: 26.06.2007 US 946281 P; 27.11.2007 US 990485 P
(43) Date of publication of application: 24.03.2010
(73) Proprietor: Massachusetts Institute of Technology, Cambridge, MA 02139-4307 (US)
(72) Inventor: TING, Alice, Allston, MA 02134 (US); BAWENDI, Moungi, Cambridge, MA 02138 (US); HOWARTH, Mark, Oxford, OX2 6BD (GB); LIU, Wenhao, Cambridge, MA 02139 (US)
(74) Representative: D Young & Co LLP
(86) International application number: PCT/US2008/067649
(87) International publication number: WO 2009/002837

(56) References cited:
- WO-A1-2006/036646
- US-A1- 2002 028 457
- US-A1- 2002 142 355
- US-A1- 2004 023 010
- US-A1- 2007 105 162
- US-A1- 2007 134 679
- US-B1- 6 326 144
- MARK HOWARTH ET AL: "Monovalent, reduced-size quantum dots for imaging receptors on living cells", NATURE METHODS, vol. 5, no. 5, 20 April 2008 (2008-04-20), pages 397-399, XP55000901, ISSN: 1548-7091, DOI: 10.1038/nmeth.1206
- H. TETSUO UYEDA ET AL: "Synthesis of Compact Multidentate Ligands to Prepare Stable Hydrophilic Quantum Dot Fluorophores", JOURNAL OF THE AMERICAN CHEMICAL SOCIETY, vol. 127, no. 11, 1 March 2005 (2005-03-01), pages 3870-3878, XP55013251, ISSN: 0002-7863, DOI: 10.1021/ja044031w
- DANIELA ZANCHET ET AL: "Electrophoretic Isolation of Discrete Au Nanocrystal/DNA Conjugates", NANO LETTERS, vol. 1, no. 1, 1 January 2001 (2001-01-01) , pages 32-35, XP55013305, ISSN: 1530-6984, DOI: 10.1021/nl005508e
- DANIELA ZANCHET ET AL: "Electrophoretic and Structural Studies of DNA-Directed Au Nanoparticle Groupings", THE JOURNAL OF PHYSICAL CHEMISTRY B, vol. 106, no. 45, 1 November 2002 (2002-11-01), pages 11758-11763, XP55013320, ISSN: 1520-6106, DOI: 10.1021/jp026144c
- FU AIHUA ET AL: "Discrete nanostructures of quantum dots/Au with DNA", JOURNAL OF THE AMERICAN CHEMICAL SOCIETY, AMERICAN CHEMICAL SOCIETY, WASHINGTON, DC; US, vol. 126, no. 35, 8 September 2004 (2004-09-08), pages 10832-10833, XP002489176, ISSN: 0002-7863, DOI: 10.1021/JA046747X
- SPERLING R A ET AL: "ELECTROPHORETIC SEPARATION OF NANOPARTICLES WITH A DISCRETE NUMBER OF FUNCTIONAL GROUPS", ADVANCED FUNCTIONAL MATERIALS, WILEY - V C H VERLAG GMBH & CO. KGAA, DE, vol. 16, no. 7, 2 May 2006 (2006-05-02), pages 943-948, XP001241762, ISSN: 1616-301X, DOI: 10.1002/ADFM.200500589 & SPERLIN ET AL.: "Supporting Material", ADV. FUNCT. MAT., vol. 16, no. 7, 2006, pages 943-948, XP007919826,
- SUSUMU K ET AL: "Enhancing the stability and biological functions of qunatum dots via compact multifuntional ligands", JOURNAL OF THE AMERICAN CHEMICAL SOCIETY, AMERICAN CHEMICAL SOCIETY, WASHINGTON, DC; US, vol. 129, no. 45, 23 October 2007 (2007-10-23), pages 13987-13996, XP002511245, ISSN: 0002-7863, DOI: 10.1021/JA0749744
- HOWARTH M ET AL: "TARGETING QUANTUM DOTS TO SURFACE PROTEINS IN LIVING CELLS WITH BIOTIN LIGASE", PROCEEDINGS OF THE NATIONAL ACADEMY OF SCIENCES OF USA, NATIONAL ACADEMY OF SCIENCE, WASHINGTON, DC; US, vol. 102, no. 21, 24 May 2005 (2005-05-24) , pages 7583-7588, XP008079740, ISSN: 0027-8424, DOI: 10.1073/PNAS.0503125102

## Description

### TECHNICAL FIELD

The invention relates to controlled modification of semiconductor nanocrystals.

### BACKGROUND

Semiconductor nanocrystals are photostable fluorophores with narrow emission spectra tunable through visible and near-infrared wavelengths, large molar extinction coefficients, and high quantum yields. These properties make semiconductor nanocrystals powerful tools for labeling and optical sensing in biological, biomedical, and environmental contexts. The exceptional brightness and photostability of semiconductor nanocrystals give them great potential for analyzing biological events at the single molecule level. However, current nanocrystals designed for cellular labeling applications suffer a tradeoff between size, non-specific binding, and derivatizability.

Nanocrystals generally include an inorganic nanoparticle that is surrounded by a layer of organic ligands. This organic ligand shell is critical to the nanocrystals for processing, binding to specific other moieties, and incorporation into various substrates. Nanocrystals can be stored in their growth solution, which contains a large excess of ligands such as alkyl phosphines and alkyl phosphine oxides, for long periods without noticeable degradation. For most applications, particularly aqueous applications, nanocrystals must be processed outside of their growth solution and transferred into various chemical environments. However, nanocrystals often lose their high fluorescence or become irreversibly aggregated when removed from their growth solution.

HOWARTH ET AL., NATURE METHODS, vol. 5, no. 5, 20 April 2008, pages 397-399, discloses a method to generate monovalent quantum dots (QDs) using agarose gel electrophoresis. The QDs are passivated with a carboxy-terminated polyethylene-glycol ligand, and then conjugated to a single copy of a high-affinity targeting moiety such as monovalent streptavidin or antibody to carcinoembryonic antigen to label cell-surface proteins.

WO 2006/036646 A1 discloses a colloidal nanoparticle bearing one or more surface ligand. The ligand includes a first module containing atoms that can attach to an inorganic surface; a second module that imparts water-solubility to the ligand and to the inorganic surface; and a third module that contains a functional group that can, directly or indirectly, conjugate to a biomolecule.

ZANCHET ET AL., NANO LETTERS, vol. 1, no. 1, 1 January 2001, pages 32-35 relates to electrophoretic isolation of 5 and 10 nm gold nanocrystals bearing discrete numbers of single-stranded DNA (1-5).

ZANCHET ET AL., THE JOURNAL OF PHYSICAL CHEMISTRY B, vol. 106, no. 45, 1 November 2002, pages 11758-11763, relates to discrete Au nanoparticle/DNA conjugates isolated by electrophoresis and used to form dimers and trimers.

SPERLING ET AL., ADVANCED FUNCTIONAL MATERIALS, vol. 16, no. 7, 2 May 2006, pages 943-948, concerns a surface functionalization of nanoparticles with a controlled number of mono- or bi-functional PEG molecules of suitable chain length. Also described are conjugates of nanoparticles with one, two or three PEG molecules per nanoparticle and their separation using gel electrophoresis, as well as the conjugation of molecules with additional functionalities to the free ends of the bifunctional PEG molecules.

US 2007/134679 A1 discloses a method for separating nanoparticles with a controlled number of active groups.

SUSUMU ET AL., JOURNAL OF THE AMERICAN CHEMICAL SOCIETY, vol. 129, no. 45, 23 October 2007, pages 13987-13996, relates to a series of modular ligands which include a PEG single chain, a dihydrolipoic acid unit and a potential biological functional group such as biotin, carboxyl and amine.

US 6,326,144 B 1 provides a composition comprising fluorescent semiconductor nanocrystals associated to a compound.

US 2007/105162 A1 relates to monovalent avidin and streptavidin compositions.

US 2004/023010 A1 discloses a light emitting device including a semiconductor nanocrystal in a layer which may be a non-polymeric layer.

US 2002/028457 A1 provides assays that allow for the detection of a single copy of a target of interest using semiconductor nanocrystal labelling and fluorescence detection.

US 2002/142355 A1 is directed to methods that can be used for biotin labelling of polypeptides in mammalian cells.

### SUMMARY

Single-particle tracking can give unprecedented understanding of the motion of cell surface proteins, free from the simplification of ensemble averaging. However, single-particle tracking is complicated by fluorophore photobleaching, probe multivalency, the size of the labeling probe, and dissociation of the probe from the target. Semiconductor nanocrystals have the intrinsic advantages for single particle tracking of brightness and photostability. Multivalency can be avoided by purifying semiconductor nanocrystals bound to a single copy of monovalent streptavidin. The size of the semiconductor nanocrystals can be comparable to that of an IgG antibody. Dissociation of the probe can be minimized by using monovalent streptavidin to link the nanocrystal to a site-specifically biotinylated cell surface protein. The monovalent semiconductor nanocrystals can be used to image, for example, neurotransmitter receptors at synapses and to follow the diffusion of wild-type or a disease-associated mutant of the low density lipoprotein receptor.

In one aspect, a method of making a controlled valency semiconductor nanocrystal includes contacting a population of semiconductor nanocrystals with a compound having an affinity for the semiconductor nanocrystal to form a distribution of compound-associated nanocrystals,; and separating the members of the distribution of compound-associated nanocrystals according to the number of compounds associated with each nanocrystal, and isolating members of the distribution of compound-associated nanocrystals which have exactly one compound associated with a nanocrystal wherein the isolated semiconductor nanocrystal is monovalent and is associated with exactly one binding site for an affinity tag or affinity target.

The compound is a monovalent avidin or a monovalent streptavidin. The compound can include a polyhistidine tag.

The semiconductor nanocrystal can include an outer layer including a compound of formula (I):

R¹-L¹-R²-L²-R³ (I)

where R¹ is a straight or branched C₁-C₁₀ alkyl, alkenyl or alkynyl chain, optionally interrupted by one or more of -O-, -S-, -C(O)-, -N(R⁴)-, or -C(O)N(R⁴)-; and substituted with two or more groups selected from hydroxy, thiol, amino, nitroxide, phosphine, or phosphine oxide. L¹ is -C(O)-, -N(R⁴)C(O)-, -C(O)N(R⁴)-, -O-, -N(R⁴)-, -O-N-(R⁴)C(O)-, -C(O)N(R⁴)-O-, or -(CR⁵R₆)ₙ-. R² is -[(CR⁵R⁶)ₙ-X-(CR⁵R⁶)ₙ]ₘ-, wherein X is O, S, C(=O), or N(R⁴); and m is an integer in the range 0 to 20. L² is -C(O)-, -N(R⁴)(O)-, -C(O)N(R⁴)-, -O-, -N(R⁴)-, -O-N(R⁴)C(O)-, -C(O)N(R⁴)-O-, or -(CR⁵R⁶)ₙ-. R³ is -(CR⁵R⁶)ₚ-R⁷ where R⁷ is -COOH, -OP(O)(OH)OH, amino, alkylamino, dialkylamino, or trialkylamino; and p is 0, 1, 2, 3, 4, 5, or 6. R⁴ is H or C₁-C₆ alkyl. Each R⁵ and each R⁶ are, independently, selected from H, hydroxy, amino, thio, nitro, alkylamino, dialkylamino, alkyl, cycloalkyl, alkenyl, alkynyl, alkoxy, aryl, and heteroaryl; and each n is independently 0, 1, 2, 3, 4, 5, or 6.

In another aspect, a method of imaging a single particle includes joining an affinity tag to a cell-surface protein; contacting the cell with a composition comprising a valency controlled semiconductor nanocrystal associated with exactly one compound having exactly one binding site for the affinity tag; wherein the semiconductor nanocrystal is associated with exactly one monovalent avidin or exactly one monovalent streptavidin; and imaging the cell and semiconductor nanorystal substantially simulataneously.

The affinity tag can be biotin. Joining an affinity tag to a cell-surface protein can include contacting a fusion protein including an acceptor peptide (AP) sequence with a biotin ligase.

The semiconductor nanocrystal may include an outer layer including a compound of formula (I) as described above.

The compound of formula (I) can have the formula: 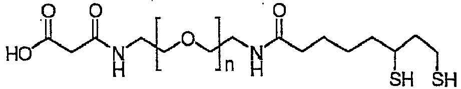 or the formula:

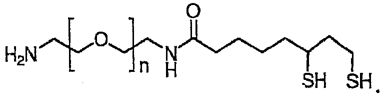

Also described herein is a semiconductor nanocrystal comprising an outer layer including compound of formula (I) as described above.

R¹ can be HS-CH₂CH₂CH(SH)-(CH₂)₄-. R² can be a poly(alkylene oxide). R² can be a poly(ethylene glycol). R² can have the formula -[CH₂-O-CH₂]ₘ-, wherein m is approximately 8. R³ can be -CH₂-R⁷ wherein R⁷ is amino, alkylamino, dialkylamino, or trialkylamino. R⁷ can be -COOH. R³ can be -CH₂COOH.

In some embodiments, R¹ is HS-CH₂CH₂CH(SH)-(CH₂)₄-, R² is a poly(alkylene oxide), and R⁷ is -COOH, amino, alkylamino, dialkylamino, or trialkylamino.

The compound can have the formula: 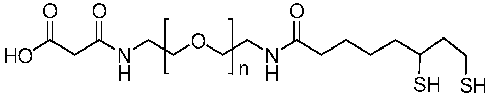 or the formula:

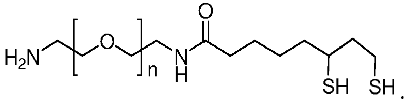

Other features, objects, and advantages of the invention will be apparent from the description and drawings, and from the claims.

### DESCRIPTION OF DRAWINGS

FIG. 1A is a graph depicting optical properties of semiconductor nanocrystals. FIG. 1B is a graph depicting physical properties of semiconductor nanocrystals.
FIG. 2A is a graph depicting electrostatic properties of semiconductor nanocrystals.
FIG. 2B is an image of an electrophoretic gel with semiconductor nanocrystals having different ligands.
FIGS. 3A-3E are fluorescence images and photographs of cells exposed to different semiconductor nanocrystals.
FIG. 4A is a schematic depiction of attachment of a dye to a semiconductor nanocrystal.
FIGS. 4B-4C is a graph depicting optical properties of semiconductor nanocrystals. FIGS. 4D-4F are graphs depicting elution profiles of standard samples and semiconductor nanocrystals during gel filtration chromatography.
FIG. 5 is a schematic depiction of labeling a cell surface protein with a semiconductor nanocrystal.
FIGS. 6A-6C are composite photograph/fluorescence images of cells. FIG. 6D is a graph depicting single molecule diffusion coefficients.
FIG. 7A is an image of an electrophoretic gel. FIGS. 7B-7E are composite photograph/fluorescence images of cells.
FIG. 8A is a schematic depiction of labeling a cell surface protein with a semiconductor nanocrystal, and detection of fluorescence via FRET. FIG. 8B are fluorescence images of cells.
FIG. 9A. is a schematic depiction of semiconductor nanocrystals having different affinity labels. FIG. 9B is a schematic depiction of labeling a single cell surface protein with a single semiconductor nanocrystal.
FIGS. 10A-10B are images of electrophoretic gel illustrating isolation of semiconductor nanocrystals associated with exactly one streptavidin.
FIG. 11A are AFM images showing semiconductor nanocrystals. FIG. 11B are fluorescence images and photographs of cells.
FIG. 12 are fluorescence images and photographs of cells.
FIG. 13 are fluorescence images of cells.

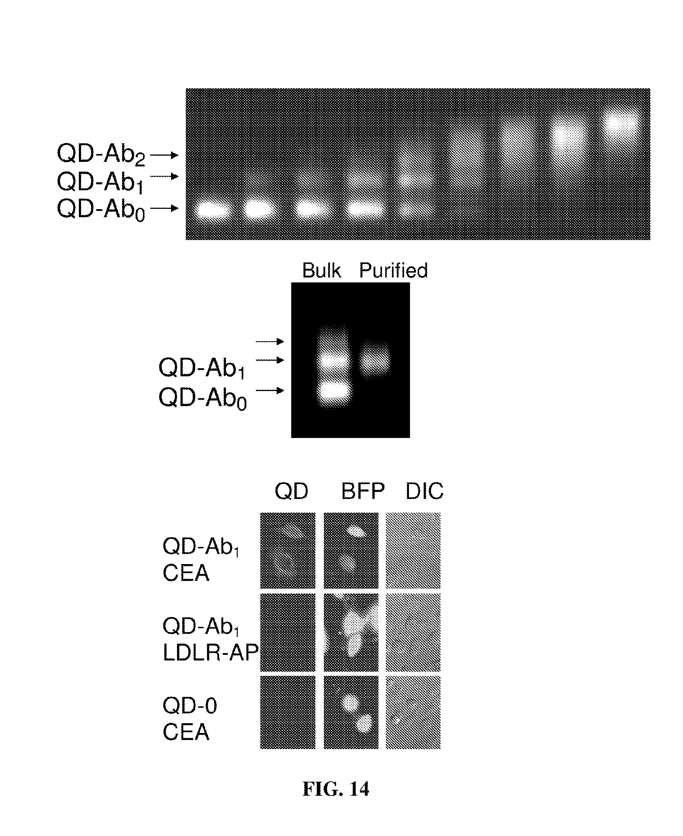

FIGS. 14A-14B are images of electrophoretic gels depicting isolation of a semiconductor nanocrystal associated with exactly one antibody. FIG. 14C are fluorescence images and photographs of cells.
FIGS. 15A-15B are fluorescence images and photographs of cells.
FIG. 16 is a schematic depiction of labeling a cell surface protein with a semiconductor nanocrystal.
FIG. 17 is a series of fluorescence images and photographs of cells.
FIG. 18 is are fluorescence images and photographs of cells.

### DETAILED DESCRIPTION

The ultimate goal of cellular imaging is to observe in living cells the movement of single biomolecules. Single molecules imaging *in vitro* has proved its ability to give the ultimate in sensitivity, detecting low abundance biomolecules such as miRNA or virus particles (see, e.g., Neely, L.A., et al.. (2006). Nat. Methods 3, 41-46; and Agrawal, A., et al. (2006). Anal. Chem. 78, 1061-1070). Single molecule imaging approaches *in vitro* also avoid the averaging inherent to ensemble methods, detecting transient conformational changes during enzymatic turnover, and kinesin steps smaller than the diffraction limit of light. See, for example, English, B.P., et al. (2006). Nat. Chem. Biol. 2, 87-94; Kusumi A., et al. (2005). Annu. Rev. Biophys. Biomol. Struct. 34, 351-378; and Yildiz, A., Selvin,P.R. (2005). Acc. Chem. Res. 38, 574-582. Single molecule imaging in cells (e.g., Saxton,M.J., Jacobson,K. (1997). Annu.Rev.Biophys.Biomal.Struct. 26, 373-399), on the other hand, is a greater challenge and a constant battle is waged against the weakness and instability of the fluorescent signal from dyes or fluorescent proteins (see, e.g., Tardin,C., et al. (2003). EMBO J. 22,4656-4665; Lommerse, P.H., et al. (2004). Biophys.J. 86, 609-616; and Douglass,A.D., Vale,R.D. (2005). Cell 121, 937-950). Dye-labeled Fab molecules are monovalent, but are dim, bleach easily and show weak binding. In a few cases single fluorescent proteins have been detected in living mammalian cells, with the sensitivity enhancement of total internal reflection fluorescence (TIRF) microscopy, but the fluorescence typically bleaches within 10 seconds. Gold particles or latex beads do allow stable single particle imaging, but are generally 30-500 nm in diameter. Gold size has recently been reduced to 5 nm by detecting not light scattering but local heating upon gold illumination (see, e.g., Lasne, D., et al. (2006). Biophys.J. 91, 4598-4604). This is a promising development but 5 nm refers to the gold core, before it was passivated and conjugated to primary and secondary antibodies.

Semiconductor nanocrystals provide certain advantages in tracking the movement of single molecules in cells. Single semiconductor nanocrystals are bright enough to be seen easily on a wide field fluorescence microscope, without the need for TIRF, and are extremely photostable (Gao, X., et al. (2005). Curr.Opin.Biotechnol. 16, 63-72). However, semiconductor nanocrystals have suffered problems of size, an unstable link between the nanocrystal and the protein of interest, and nanocrystal multivalency (i.e., the presence of multiple target-binding sites on the nanocrystal, such that the nanocrystal can cross-link multiple targets). See, for example, Groc, L., et al. (2004). Nat.Neurosci. 7, 695-696; Howarth, M., et al. (2005). Proc.Natl.Acad Sci. U.S.A. 102, 7583-7588; and Jaiswal, J. K. and Simon, S: M. (2004) Trends Cell Biol. 14[9], 497-504. Nanocrystals in common use are 20-30 nm in diameter, which may impair diffusion in crowded cellular locations such as the cytosol or synapses. An unstable link between the nanocrystal and the target protein results from monovalent antibodies dissociating from their targets typically on the order of minutes, which is too short to track faithfully most dynamic process in living cells (see, e.g., Schwesinger, F., et al. (2000). Proc.Natl.AcadSci.U.SA. 97, 9972-9977).

Multivalency of nanocrystals (as well as of other nanoparticles, such as gold nanoparticles) is a grave concern: cross-linking of surface proteins can activate signaling pathways and can lower receptor mobility by promoting contact with the cytoskeleton. See, for example, Klemm, J.D., et al. (1998). Annu.Rev.Immunol. 16, 569-592; and Iino, R., et al. (2001). Biophys.J. 80, 2667-2677). Adding nanocrystals in excess does not fully address the cross-linking problem, since it is possible for the target protein to dissociate from one nanocrystal and cross-link to another nanocrystal. Alternatively, pools of the target protein, for example in recycling endosomes, that are not accessible to the initial pulse of nanocrystal may reach the surface and cross-link to free binding sites on another nanocrystal. A complete solution to potential cross-linking requires nanoparticles that uniformly bear a single binding site.

One approach to prepare monovalent nanoparticles is based upon electrophoretic separation via the mobility shift from different numbers of DNA ligands. See, for example, Fu, A., et al. (2004). J.Am.Chem.Soc. 126, 10832-10833; Qin, W.J., Yung, L.Y. (2005). Langmuir 21, 11330-11334; and Ackerson, C.J., et al. (2005). Proc.Natl.Acad.Sci.U.S.A. 102, 13383-13385. Methods to control protein valency based on DNA would require subsequent removal of the DNA if the nanoparticle is not to be very large. An alternative approach is based on the low density of functional sites on a polystyrene bead, allowing one ligand on a particle to be activated for ligand attachment (see, for example, Sung, K.M., et al. (2004). J.Am.Chem.Soc. 126, 5064-5065; and Worden, J.G., et al. (2004). Chem. Commun. (Camb.) 518-519). This method only gave ~60% monovalency. Purification of gold nanoparticles according to the number of attached His₆-tagged peptides has been demonstrated using a nickel affinity column, but there was significant overlap in the elution profile of monovalent and multivalent particles (Levy, R., et al. (2006). Chembiochem. 7, 592-594). Another approach has been to add a ligand such as biotinylated epidermal growth factor (EGF) to streptavidin-nanocrystals at sub-stoichiometric ratios, such that most nanocrystals bind no EGF, some bind one EGF, and almost none bind two EGF molecules (Lidke, D.S., et al. (2005). J.Cell Biol. 170, 619-626). This is an inefficient use of nanocrystals, and nanocrystals can cross-link to each other if the biotinylated ligand contains more than one biotin group.

The optimal design of semiconductor nanocrystals for single molecule imaging on live cells presents a unique set of challenges. The nanoparticle should feature facile derivatization via multiple conjugation strategies, low non-specific binding, small hydrodynamic size, high quantum yield, and the ability to form aggregate-free aqueous dispersions across a wide pH range.

Presently, the dominant class of nanocrystals used for single molecule cellular imaging are those which retain hydrophobic surface ligands from synthesis and are encapsulated in amphiphilic polymer shells. See, for example, Wu, X.; et al., Nature Biotechnol. 2003,21,41-46; Medintz, I.; et al., Nature Mater. 2005, 4, 435-446; Zhou, M.; et al., Bioconjugate Chem. 2007, 18, 323-332; Dahan, M.; et al., **2003**, 302, 442-445; and Courty, S.; et al, Nano. Lett. 2006, 6, 1491-1495. Such encapsulated nanocrystals benefit from high quantum yield (QY), but the polymeric shell produces large assemblies on the order of 20-30 nm for an inorganic core/shell diameter of only 4-6 nm (see, e.g., Smith, A. M.; et al., Phys. Chem. Chem. Phys. 2006, 8, 3895-3903). The size of polymer-coated nanocrystals, which is often much larger than the targets being labeled, has been a major barrier to the widespread use of nanocrystals in biological imaging, potentially interfering with the function of labeled proteins and limiting access to hindered spaces such as neuronal synapses. See, for example, Howarth, M.; et al., PNAS. 2005, 102, 7583-7588; and Groc, L.; et al., Nat Neurosci 2004, 7, 695-696. Furthermore, amphiphilic polymer coatings are often highly charged, which contributes to non-specific binding to cell membranes, rendering them unsuitable for single-particle imaging where low background is essential. Non-specific adsorption can be mitigated via PEGylation of polymer-encapsulated nanocrystals, but this further increases nanoparticle size (see, e.g., Bentzen, E. L.; et al., Bioconjugate Chem. 2005, 16, 1488-1494). Nanocrystals coated with phospholipids or silica shells have also been used in biological systems but suffer from similar limitations of inherently large size and the need for a bulky PEG passivating layer. See, for example, Dubertret, B.; et al., Science 2002, 298, 1759-1762; and Parak, W. J.; et al., 2002, 14, 2113-2119.

The size of nanocrystals can be dramatically reduced, while maintaining derivatizability, by displacing the native hydrophobic coating with carboxylate-bearing small molecule coordinating ligands such as mercaptoacetic acid (MAA). See, e.g., Aldana, J.; et al., J. Am. Chem. Soc. 2001, 123, 8844-8850; Mattoussi, H.; et al., J. Am. Chem. Soc. 2000, 122, 12142-12150; Kim, S.; Bawendi, M. G., J. Am. Chem. Soc. 2003, 125, 14652-14653; and Algar, W. R; Krull, U. J., 2006, 22, 11346-11352). Although such nanocrystals have hydrodynamic diameters (HDs) of only ~6-8 nm, they can be inherently unstable due to weak ligand-nanocrystal interactions, leading to nanocrystal precipitation on the time scale of several hours under ambient conditions. See, for example, Smith, A. M.; et al., Phys. Chem. Chem. Phys. 2006, 8, 3895-3903; and Aldana, J.; et al., J. Am. Chem. Soc. 2001, 123, 8844-8850. In addition, the ionization of the carboxylate group required to render the nanocrystals water dispersable results in instability under acidic conditions and also promotes non-specific binding to cells (Bentzen, E. L.; et al., Bioconjugate Chem. 2005, 16, 1488-1494; and Xue, F.; et al., Journal of Fluorescence 2007, 17, 149-154). Moreover, nanocrystals ligand-exchanged with such mono-thiol based ligands typically suffer a dramatic decrease in quantum yield (Smith, A. M.; et al., Phys. Chem. Chem. Phys. 2006, 8, 3895-3903). Dithiol ligands, such as dihydrolipoic acid (DHLA), are much more stable with respect to ligand dissociation, but still yield nanocrystals that precipitate under weakly acidic conditions. See, for example, Mattoussi, H.; et al, J. Am. Chem. Soc. 2000, 122, 12142-12150; and Pons, T.; et al, J. Phys. Chem. B 2006, 110, 20308-20316. Furthermore, DHLA coated nanocrystals exhibit high non-specific binding, rendering them unusable for single particle tracking applications. Ligand exchange with esters of DHLA with various length PEGs yielded nanocrystals that were highly stable in aqueous solution and suitable for live cell imaging (see, e.g, Uyeda, H. T.; et al., J. Am. Chem. Soc. 2005, 127, 3870-3878). However, the hydroxyl-terminated surface of these DHLA-PEG nanocrystals lacks the functionality for efficient and selective covalent derivatization under mild conditions, for example with targeting biomolecules for receptor labeling on cells.

Two commonly employed nanocrystal derivatization strategies are direct covalent modification of nanocrystals using common bioconjugation methods such as 1-ethyl-3-(3-dimethylaminopropyl) carbodiimide (EDC) and N-hydroxysuccinimide (NHS) mediated cross-coupling between amino and carboxyl functionalities, and self-assembly of biomolecules onto nanocrystals via electrostatic or metal-affinity (such as His-tag) interactions. See, for example, Wu, X.; et al., Nature Biotechnol. 2003, 21, 41-46; Yan Zhang; et al., Angew. Chem. Int. Ed. 2006, 45, 4936-4940; and Goldman, E. R.; et al., J. Am. Chem. Soc. 2002, 124, 6378-6382. Nanocrystals encapsulated in polymeric/phospholipid/silica shells are generally derivatized by covalent conjugation. Nanocrystals capped with DHLA or DHLA-PEG are amenable to conjugation using metal-affinity interactions between a His₆-tagged biomolecule and the metal surface of the nanocrystal, leading to stable conjugates that retain both nanocrystal luminescence and functionality of the coordinated biomolecules (see, e.g., Medintz, I. L., Nature Mater. 2003, 2, 630-638). Presently, there is the lack of a robust strategy which combines the ability for covalent and His₆-tag conjugation on a single nanoparticle, while maintaining the properties of small size, low non-specific binding, and solution stability.

A nanocrystal ligand can include a first portion that includes one or more coordinating atoms, a second portion including one or more hydrophilic groups, and a third portion including one or more ionizable groups. The coordinating atoms can be, for example, N, O, P, or S. The coordinating atoms can be included in a coordinating group, e.g., an amine, a nitroxide, an alcohol, a carboxylate, a thiocarboxylate, a phosphine, a phosphine oxide, a thiol, or the like. The hydrophilic groups can include, for example, an alkoxide, an amine, a thiol, an alcohol, a carboxylate, a ketone, or an aldehyde. The ionizable group can be a group that has an electrostatic charge or one that can become electrostatically charged in an aqueous environment. Exemplary ionizable groups include amines (e.g., primary, secondary, tertiary and quaternary amines), carboxylates, alcohols, and thiols.

In some embodiments, the first portion, second portion and third portion of the ligand are each derived from a separate precursor compound. The first portion can include more than one coordinating atom, such as 2, 3, 4, 5, 6 or more coordinating atoms. In some embodiments, the first portion has 2 coordinating atoms. The second portion can be substantially derived from a poly(ethylene glycol) or poly(propylene glycol).

R¹-L¹-R²-L²-R³ (I)

where R¹ is a straight or branched C₁-C₁₀ alkyl, alkenyl or alkynyl chain, optionally interrupted by one or more of -O-, -S-, -C(O)-; -N(R⁴)-, or -C(O)N(R⁴)-; and substituted with two or more groups selected from hydroxy, thiol, amino, nitroxide, phosphine, or phosphine oxide.

L¹ is -C(O)-, -N(R⁴)C(O)-, -C(O)N(R⁴)-, -O-, -N(R⁴)-, -O-N(R⁴)C(O)-, -C(O)N(R⁴)-O-, or -(CR⁵R⁶)ₙ-.

R² is -[(CR⁵R⁶)ₙ-X-(CR⁵R⁶)ₙ]ₘ-, wherein X is O, S, C(=O), or N(R⁴); and m is an integer in the range 0 to 20.

L² is -C(O)-, -N(R⁴)C(O)-, -C(O)N(R⁴)-, -O-, -N(R⁴)-, -O-N(R⁴)C(O)-, -C(O)N(R⁴)-O-, or -(CR⁵R⁶)ₙ-.

R³ is -(CR⁵R⁶)ₚ-R⁷ where R⁷ is -COOH, -OP(O)(OH)OH, amino, alkylamino, dialkylamino, or trialkylamino; and p is 0, 1, 2, 3, 4, 5, or 6.

R⁴ is H or C₁-C₆ alkyl.

Each R⁵ and each R⁶ are, independently, selected from H, hydroxy, amino, thio, nitro, alkylamino, dialkylamino, alkyl, cycloalkyl, alkenyl, alkynyl, alkoxy, aryl, and heteroaryl.

Each n is independently 0, 1, 2, 3, 4, 5, or 6.

In some embodiments, the ligand can have the formula: 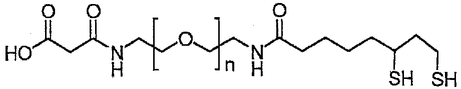 where n is in the range 1 to 100, 1 to 50, or 1 to 20. The value of n can be approximately 8. In other words, while an individual molecule of the ligand can have only integer values, in a bulk sample of the ligand, n can have a distribution of integer values, where the mean value of n is approximately 8.

An efficient route to nanocrystals coated with DHLA-PEG, terminating in amine or carboxy functional groups is described. These nanocrystals have good photophysical properties and a size substantially smaller than encapsulated nanocrystals. These nanocrystals can be conjugated via both covalent bond formation and His₆-protein coupling for targeted cell labeling applications. The quantum yield (QY) of these DHLA-PEG derivatized nanocrystals was enhanced via an alloyed ZnCd₁₋ₓSₓ shell, to give a QY after ligand exchange as high as 45%. These nanocrystals exhibited very low non-specific binding to cells and have good pH stability. The nanocrystals were also used for live cell imaging by targeting the low density lipoprotein receptor, a protein whose trafficking has an important role in preventing coronary artery disease. In addition, the use of biotin ligase for nanocrystal targeting, is described by using a monovalent variant of streptavidin as a high affinity and low valency bridge to firmly link nanocrystals to specifically biotinylated receptors (see, e.g., Howarth, M.; et al., PNAS. 2005,102, 7583-7588; and Howarth, M.; et al., Nat Meth 2006, 3, 267-273).

Nanocrystal cores can be prepared by the pyrolysis of organometallic precursors in hot coordinating agents. See, for example, Murray, C.B., et al., J. Am. Chem. Soc. 1993, 115, 8706, and Mikulec, F., Ph.D. Thesis, MIT, Cambridge, 1999. Growth of shell layers on the bare nanocrystal cores can be carried out by simple modifications of conventional overcoating procedures. See, for example, Peng, X., et al., J. Am. Chem. Soc. 1997, 119, 7019, Dabbousi, B.O., et al., J. Phys. Chem. B 1997, 101, 9463, and Cao, Y. W. and Banin, U. Angew. Chem. Int. Edit. 1999, 38, 3692.

A coordinating agent can help control the growth of the nanocrystal. The coordinating agent is a compound having a donor lone pair that, for example, has a lone electron pair available to coordinate to a surface of the growing nanocrystal. The coordinating agent can be a solvent. A coordinating agent can stabilize the growing nanocrystal. Typical coordinating agents include alkyl phosphines, alkyl phosphine oxides, alkyl phosphonic acids, or alkyl phosphinic acids, however, other coordinating agents, such as pyridines, furans, and amines may also be suitable for the nanocrystal production. Examples of suitable coordinating agents include pyridine, tri-n-octyl phosphine (TOP) and tri-n-octyl phosphine oxide (TOPO). Technical grade TOPO can be used.

The outer surface of the nanocrystal can include a layer of compounds derived from the coordinating agent used during the growth process. The surface can be modified by repeated exposure to an excess of a competing coordinating group to form an overlayer. For example, a dispersion of nanocrystals capped with the coordinating agent used during growth can be treated with a coordinating organic compound, such as pyridine, to produce crystallites which disperse readily in pyridine, methanol, and aromatics but no longer disperse in aliphatic solvents. Such a surface exchange process can be carried out with any compound capable of coordinating to or bonding with the outer surface of the nanocrystal, including, for example, phosphines, thiols, amines and phosphates. The nanocrystal can be exposed to short chain polymers which exhibit an affinity for the surface and which terminate in a moiety having an affinity for a suspension or dispersion medium. Such affinity improves the stability of the suspension and discourages flocculation of the nanocrystal.

Monodentate alkyl phosphines and alkyl phosphine oxides passivate nanocrystals efficiently. Note that the term phosphine will refer to both phosphines and phosphine oxides below. Other conventional ligands such as thiols or phosphonic acids can be less effective than monodentate phosphines for maintaining the initial high nanocrystal luminescence over long periods. For example, the photoluminescence of nanocrystals consistently diminishes or quenches after ligand exchanges with thiols or phosphonic acid.

Ligand exchanges can be carried out by one-phase or two-phase methods. Prior to ligand exchange, nanocrystals can be precipitated from their growth solutions by addition of methanol. The supernatant solution, which includes excess coordinating agent (e.g., trioctylphosphine), can be discarded. The precipitated nanocrystals can be redispersed in hexanes. Precipitation and redispersion can be repeated until essentially all the excess coordinating agent has been separated from the nanocrystals. A one-phase process can be used when both the nanocrystals and the ligands to be introduced are soluble in the same solvent. A solution with an excess of new ligands can be mixed with the nanocrystals. The mixture can be stirred at an elevated temperature until ligand exchange is complete. The one-phase method can be used, for example, to exchange octyl-modified oligomeric phosphines or methacrylate-modified oligomeric phosphines, which are both soluble in solvents that are compatible with the nanocrystals, such as hexanes. A two-phase ligand exchange process can be preferable when the nanocrystals and the new ligands do not have a common solvent. Nanocrystals can dissolved in an organic solvent such as dichloromethane, and the new ligand can be dissolved in an aqueous solution. The nanocrystals can be transferred from the organic phase to the aqueous phase by, for example, sonication. The transfer can be monitored through absorption and emission spectroscopy. similar two-phase ligand exchange process has been reported earlier. See, for example, Wang, Y.A., et al., 2002 J. Am. Chem. Soc 124,2293.

The nanocrystal can be a member of a population of nanocrystals having a narrow size distribution. The nanocrystal can be a sphere, rod, disk, or other shape. The nanocrystal can include a core of a semiconductor material. The nanocrystal can include a core having the formula MX, where M is cadmium, zinc, magnesium, mercury, aluminum, gallium, indium, thallium, or mixtures thereof, and X is oxygen, sulfur, selenium, tellurium, nitrogen, phosphorus, arsenic, antimony, or mixtures thereof.

The semiconductor forming the core of the nanocrystal can include Group II-VI compounds, Group II-V compounds, Group III-VI compounds, Group III-V compounds, Group IV-VI compounds, Group I-III-VI compounds, Group II-IV-VI compounds, and Group II-IV-V compounds, for example, ZnS, ZnSe, ZnTe, CdS, CdSe, CdTe, HgS, HgSe, HgTe, AlN, AlP, AlAs, AlSb, GaN, GaP, GaAs, GaSb, GaSe, InN, InP, InAs, InSb, TIN, TlP, TlAs, TlSb, PbS, PbSe, PbTe, or mixtures thereof.

The core can have an overcoating on a surface of the core. The overcoating can be a semiconductor material having a composition different from the composition of the core. The overcoat of a semiconductor material on a surface of the nanocrystal can include a Group II-VI compounds, Group II-V compounds, Group III-VI compounds, Group III-V compounds, Group IV-VI compounds, Group I-III-VI compounds, Group II-IV-VI compounds, and Group II-IV-V compounds, for example, ZnS, ZnSe, ZnTe, CdS, CdSe, CdTe, HgS, HgSe, HgTe, AlN, AlP, AlAs, AlSb, GaN, GaP, GaAs, GaSb, GaSe, InN, InP, InAs, InSb, TIN, TlP, TlAs, TlSb, PbS, PbSe, PbTe, or mixtures thereof. The overcoating material can have a band gap greater than the band gap of the core material. Alternatively, the overcoating material can have a band (i.e. the valence band or the conduction band) intermediate in energy to the valence and conduction bands of the core material. See for example, U.S. Patent Application Publication No. 20040110002 titled, "Semiconductor Nanocrystal Heterostructures".

The emission from the nanocrystal can be a narrow Gaussian emission band that can be tuned through the complete wavelength range of the ultraviolet, visible, or infrared regions of the spectrum by varying the size of the nanocrystal, the composition of the nanocrystal, or both. For example, CdSe can be tuned in the visible region and InAs can be tuned in the infrared region.

The population of nanocrystals can have a narrow size distribution. The population can be monodisperse and can exhibit less than a 15% rms deviation in diameter of the nanocrystals, preferably less than 10%, more preferably less than 5%. Spectral emissions in a narrow range of between 10 and 100 nm full width at half max (FWHM) can be observed. Semiconductor nanocrystals can have emission quantum efficiencies of greater than 2%, 5%, 10%, 20%, 40%, 60%, 70%, or 80%.

Methods of preparing semiconductor nanocrystals include pyrolysis of organometallic reagents, such as dimethyl cadmium, injected into a hot, coordinating agent. This permits discrete nucleation and results in the controlled growth of macroscopic quantities of nanocrystals. Preparation and manipulation of nanocrystals are described, for example, in U.S. Patent No..6,322,901. The method of manufacturing a nanocrystal is a colloidal growth process and can produce a monodisperse particle population. Colloidal growth occurs by rapidly injecting an M donor and an X donor into a hot coordinating agent The injection produces a nucleus that can be grown in a controlled manner to form a nanocrystal. The reaction mixture can be gently heated to grow and anneal the nanocrystal. Both the average size and the size distribution of the nanocrystals in a sample are dependent on the growth temperature. The growth temperature necessary to maintain steady growth increases with increasing average crystal size. The nanocrystal is a member of a population of nanocrystals. As a result of the discrete nucleation and controlled growth, the population of nanocrystals obtained has a narrow, monodisperse distribution of diameters. The monodisperse distribution of diameters can also be referred to as a size. The process of controlled growth and annealing of the nanocrystals in the coordinating agent that follows nucleation can also result in uniform surface derivatization and regular core structures. As the size distribution sharpens, the temperature can be raised to maintain steady growth. By adding more M donor or X donor, the growth period can be shortened.

An overcoating process is described, for example, in U.S. Patent No. 6,322,901. By adjusting the temperature of the reaction mixture during overcoating and monitoring the absorption spectrum of the core, over coated materials having high emission quantum efficiencies and narrow size distributions can be obtained.

The M donor can be an inorganic compound, an organometallic compound, or elemental metal. The inorganic compound M-containing salt can be a metal halide, metal carboxylate, metal carbonate, metal hydroxide, or metal diketonate, such as a metal acetylacetonate. See, for example, U.S. Patent No. 6,576,291. M is cadmium, zinc, magnesium, mercury, aluminum, gallium, indium or thallium. The X donor is a compound capable of reacting with the M donor to form a material with the general formula MX. Topically, the X donor is a chalcogenide donor or a pnictide donor, such as a phosphine chalcogenide, a bis(silyl) chalcogenide, dioxygen, an ammonium salt, or a tris(silyl) pnictide. Suitable X donors include dioxygen, bis(trimethylsilyl) selenide ((TMS)₂Se), trialkyl phosphine selenides such as (tri-n-octylphosphine) selenide (TOPSe) or (tri-n-butylphosphine) selenide (TBPSe), trialkyl phosphine tellurides such as (tri-n-octylphosphine) telluride (TOPTe) or hexapropylphosphorustriamide telluride (HPPTTe), bis(trimethylsilyl)telluride ((TMS)₂Te), bis(trimethylsilyl)sulfide ((TMS)₂S), a trialkyl phosphine sulfide such as (tri-n-octylphosphine) sulfide (TOPS), an ammonium salt such as an ammonium halide (e.g., NH₄Cl), tris(trimethylsilyl) phosphide ((TMS)₃P), tris(trimethylsilyl) arsenide ((TMS)₃As), or tris(trimethylsilyl) antimonide ((TMS)₃Sb). In certain embodiments, the M donor and the X donor can be moieties within the same molecule.

Size distribution during the growth stage of the reaction can be estimated by monitoring the absorption line widths of the particles. Modification of the reaction temperature in response to changes in the absorption spectrum of the particles allows the maintenance of a sharp particle size distribution during growth. Reactants can be added to the nucleation solution during crystal growth to grow larger crystals. By stopping growth at a particular nanocrystal average diameter, a population having an average nanocrystal diameter of less than 150 Å can be obtained, A population of nanocrystals can have an average diameter of 15 Å to 125 Å.

The particle size distribution can be further refined by size selective precipitation with a poor solvent for the nanocrystals, such as methanol/butanol as described in U.S. Patent No. 6,322,901. For example, nanocrystals can be dispersed in a solution of 10% butanol in hexane. Methanol can be added dropwise to this stirring solution until opalescence persists. Separation of supernatant and flocculate by centrifugation produces a precipitate enriched with the largest crystallites in the sample. This procedure can be repeated until no further sharpening of the optical absorption spectrum is noted. Size-selective precipitation can be carried out in a variety of solvent/nonsolvent pairs, including pyridine/hexane and chloroform/methanol. The size-selected nanocrystal population can have no more than a 15% rms deviation from mean diameter, preferably 10% rms deviation or less, and more preferably 5% rms deviation or less.

Transmission electron microscopy (TEM) can provide information about the size, shape, and distribution of the nanocrystal population. Powder X-ray diffraction (XRD) patterns can provided the most complete information regarding the type and quality of the crystal structure of the nanocrystals. Estimates of size are also possible since particle diameter is inversely related, via the X-ray coherence length, to the peak width. For example, the diameter of the nanocrystal can be measured directly by transmission electron microscopy or estimated from X-ray diffraction data using, for example, the Scherrer equation. It also can be estimated from the UV/Vis absorption spectrum.

A controlled-valency semiconductor nanocrystal can have a desired valency with respect to binding sites for affinity targets. Thus a semiconductor nanocrystal is prepared that has exactly one,

binding site for an affinity target. A population of semiconductor nanocrystals can be prepared in which each semiconductor nanocrystal in the population has exactly a desired valency. A monovalent semiconductor nanocrystal has exactly one binding site for an affinity target

A controlled-valency semiconductor nanocrystal is prepared by linking a compound to a semiconductor nanocrystal. In some embodiments, the linking produces a stochastic distribution of numbers of compounds bound to individual semiconductor nanocrystals. The distribution can be influenced by altering the relative quantities of compound and semiconductor nanocrystal present during linking. Members of the distribution having different numbers of compounds (such as, for example, zero compounds, exactly one compound, exactly two compounds, exactly three compounds, or exactly a higher number of compounds) bound to the semiconductor nanocrystal are separated from one another. The separation can be a size-based separation (such as, for example, electrophoresis or gel filtration chromatography), or an affinity-based separation (such as, for example, affinity chromatography). Affinity chromatography can include generating a column bearing immobile ligands (i.e., affinity targets) to which the compound binds. The affinity targets can be linked to a chromatography resin bearing reactive functional groups according to methods known in the art. Nanocrystals associated with the compound can become immobilized with the resin by virtue of the interaction between the compound and the affinity target Nanocrystals not associated with the column are not immobilized and can be efficiently removed by washing the resin. Subsequently, the resin is washed with an elution condition (e.g., a change in pH, ionic strength, temperature, or exposure to a competing ligand or ligand analog) that weakens the interaction between the compound and the nanocrystal. Increasing exposure to the elution condition further weakens the interaction, such that nanocrystals associated with exactly one compound will elute from the resin before nanocrystals having exactly compounds associated, and so on.

This interaction could either be a specific binding interaction, such as an antibody binding its target antigen, or maltose binding protein binding to amylase; an electrostatic interaction such as a highly charged peptide binding to a cation or anion exchange column; or a metal-ligand interaction like a nickel or cobalt column binding to polyimidazole tags (e.g., a His₆ tag).

In some embodiments, a population of semiconductor nanocrystals can be prepared, in which substantially all of the semiconductor nanocrystals are linked to exactly one compound. In some embodiments, the compound has exactly one binding site, exactly two binding sites, exactly three binding sites, or exactly four binding sites for an affinity target In this way, a population of semiconductor nanocrystals can be prepared in which each member of the population is associated with an exact number ranging from zero to twelve or more of binding sites for an affinity target.

The compound is a monovalent avidin (mA) or a monovalent streptavidin (mSA).

The affinity target can be, for example, a protein, a nucleic acid, a peptide, a metabolite, or a small molecule.

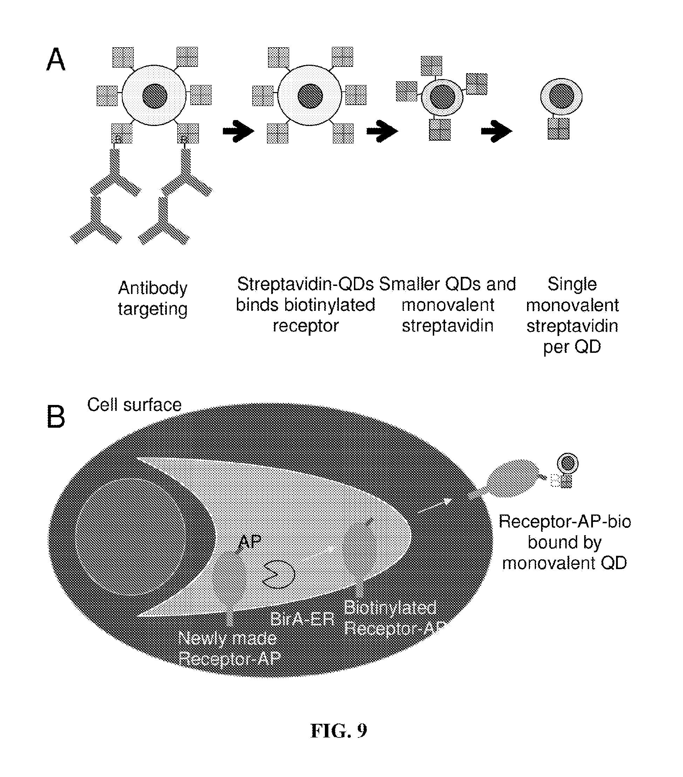

Cell surface proteins can be labeled with nanocrystals by antibody targeting (FIG. 9A). A method to site-specifically biotinylate cell surface proteins using biotin ligase is known (see, for example, Howarth, M., et al. (2005). Proc.Natl.Acad.Sci. U.S.A. 102, 7583-7588; and Chen, I., et al. (2005). Nat.Methods 2, 99-104). Biotin ligase rapidly biotinylates a lysine sidechain within a 15-amino acid acceptor peptide (AP) sequence, genetically added to the protein of interest. See, for example, Beckett, D., et al. Protein Sci. 1999 Apr;8(4):921-9. The biotinylated AP-tagged proteins can then be stably tracked at the single-molecule level using streptavidin-coated nanocrystals (Howarth, M., et al. 2005). The high stability of streptavidin labeling avoids the reversibility on the order of minutes which is characteristic of most antibody-antigen interactions (Green, N.M. (1990). Methods Enzymol. 184, 51-67). However, the size and multivalency of nanocrystals used in previous work are barriers to obtaining reliable quantitative data on receptor diffusion and trafficking (see, e.g., Groc, L., (2004). Nat.Neurosci. 7, 695-696; and Howarth, M., et al. (2005). Proc.Natl.AcadSci.U.SA. 102, 7583-7588). Commercial streptavidin-nanocrystals have 4-10 copies of tetravalent streptavidin per particle (see, for example, Grecco, H.E. et al. (2004). Microsc.Res.Tech. 65, 169-179). This gives 16-40 potential biotin binding sites per nanocrystal. In addition, up to 30% of certain commercial nanocrystals may be dimerized or trimerized (Nehilla,B.J., et al. (2005). J.Phys.Chem.B Condens.Matter Mater.Surf.Interfaces.Biophys. 109, 20724-20730).

To avoid cross-linking when labeling cell surface proteins with tetrameric streptavidin, a streptavidin bearing one, rather than four, biotin binding sites has been developed (see, e.g., Howarth, M., et al. (2006). Nat.Methods 3, 267-273; and U.S. Patent Application Publication No. 2007/0099248, titled "Monovalent streptavidin compositions"). This streptavidin had the same binding affinity, off-rate and thermostability as wild-type streptavidin, but did not cause cross-linking when used to label cell surface proteins (Howarth *et al*., 2006).

A modified streptavidin monomer can have a modification of the core wild-type streptavidin amino acid sequence. A modification of a sequence of a streptavidin subunit is a change in the amino acid sequence of the streptavidin monomer subunit from the wild-type amino acid sequence. Modifications of a streptavidin amino acid sequence may include the substitution of one or more amino acid residues in the sequence for alternative amino acids, A substitution of one amino acid for another in the mature sequence of wild-type streptavidin (residues 25-163 of SEQ ID NO:1), is an example of a modification of a streptavidin subunit. Residue 25 of the sequence set forth as Genbank Accession No. P22629 (SEQ ID NO:1) is considered to be residue one of mature wild-type streptavidin monomer and residue 37 of SEQ ID NO:1 is considered to be residue one of wild-type core streptavidin sequence. Using this numbering system the residues that are altered in the preparation of some modified monomers of the invention include residues N23, S27, and S45. An example of a modified streptavidin monomer subunit is a D subunit, which includes the following substitutions: N -> A at position 23 in the amino acid sequence of mature wild-type streptavidin monomer; S -> D at position 27 in the amino acid sequence of mature wild-type streptavidin monomer; and S -> A at position 45 in the amino acid sequence of mature wild-type streptavidin monomer.

### Wild-type streptavidin:

Met Arg Lys Ile Val Val Ala Ala Ile Ala Val Ser Leu Thr Thr Val
1               5                   10                  15

Ser Ile Thr Ala Ser Ala Ser Ala Asp Pro Ser Lys Asp Ser Lys Ala
            20                  25                  30

Gln Val Ser Ala Ala Glu Ala Gly Ile Thr Gly Thr Trp Tyr Asn Gln
        35                  40                  45

Leu Gly Ser Thr Phe Ile Val Thr Ala Gly Ala Asp Gly Ala Leu Thr
    50                  55                  60

Gly Thr Tyr Glu Ser Ala Val Gly Asn Ala Glu Ser Arg Tyr Val Leu
65                  70                  75                  80

Thr Gly Arg Tyr Asp Ser Ala Pro Ala Thr Asp Gly Ser Gly Thr Ala
                85                  90                  95

Leu Gly Trp Thr Val Ala Trp Lys Asn Asn Tyr Arg Asn Ala His Ser
            100                 105                 110

Ala Thr Thr Trp Ser Gly Gln Tyr Val Gly Gly Ala Glu Ala Arg Ile
        115                 120                 125

Asn Thr Gln Trp Leu Leu Thr Ser Gly Thr Thr Glu Ala Asn Ala Trp
    130                 135                 140

Lys Ser Thr Leu Val Gly His Asp Thr Phe Thr Lys Val Lys Pro Ser
145                 150                 155                 160

Ala Ala Ser Ile Asp Ala Ala Lys Lys Ala Gly Val Asn Asn Gly Asn
                165                 170                 175

Pro Leu Asp Ala Val Gln Gln
            180

The sequence set forth as the Dead (D) monomer sequence includes the following substituted amino acid residues: N23A, S27D, and S45A (with the numbering based on the numbering of the mature wild-type streptavidin sequence, which corresponds to amino acids 1-163 of SEQ ID NO:1). A Dead monomer sequence is set forth herein as SEQ ID NO:3. The sequence of the Alive (A) monomer subunit, which as described above has the unmodified core wild-type streptavidin monomer sequence and may also include a His₆ purification tag. In some embodiments, an Alive (A) monomer does not have a purification tag. The Alive streptavidin monomer subunit with a His₆ tag is set forth herein as SEQ ID NO:4. For use in some methods and preparations of the invention, the sequences set forth as SEQ ID NO:2, 3, and 4 are encoded in a plasmid with an initiating methionine, which is then removed by the E. coli. It will be understood that the presence of an initiating methionine is not an alteration of the core sequence thus is not a modification.

### Dead Monomer:

Ala Glu Ala Gly Ile Thr Gly Thr Trp Tyr Ala Gln Leu Gly Asp Thr
1               5                   10                  15

Phe Ile Val Thr Ala Gly Ala Asp Gly Ala Leu Thr Gly Thr Tyr Glu
            20                  25                  30

Ala Ala Val Gly Asn Ala Glu Ser Arg Tyr Val Leu Thr Gly Arg Tyr
        35                  40                  45

Asp Ser Ala Pro Ala Thr Asp Gly Ser Gly Thr Ala Leu Gly Trp Thr
    50                  55                  60

Val Ala Trp Lys Asn Asn Tyr Arg Asn Ala His Ser Ala Thr Thr Trp
65                  70                  75                  80

Ser Gly Gln Tyr Val Gly Gly Ala Glu Ala Arg Ile Asn Thr Gln Trp
                85                  90                  95

Leu Leu Thr Ser Gly Thr Thr Glu Ala Asn Ala Trp Lys Ser Thr Leu
            100                 105                 110

Val Gly His Asp Thr Phe Thr Lys Val Lys Pro Ser Ala Ala Ser
        115                 120                 125

### Alive monomer:

Ala Glu Ala Gly Ile Thr Gly Thr Trp Tyr Asn Gln Leu Gly Ser Thr
1               5                   10                  15

Phe Ile Val Thr Ala Gly Ala Asp Gly Ala Leu Thr Gly Thr Tyr Glu
            20                  25                  30

Ser Ala Val Gly Asn Ala Glu Ser Arg Tyr Val Leu Thr Gly Arg Tyr
        35                  40                  45

Asp Ser Ala Pro Ala Thr Asp Gly Ser Gly Thr Ala Leu Gly Trp Thr
    50                  55                  60

Val Ala Trp Lys Asn Asn Tyr Arg Asn Ala His Ser Ala Thr Thr Trp
65                  70                  75                  80

Ser Gly Gln Tyr Val Gly Gly Ala Glu Ala Arg Ile Asn Thr Gln Trp
                85                  90                  95

Leu Leu Thr Ser Gly Thr Thr Glu Ala Asn Ala Trp Lys Ser Thr Leu
            100                 105                 110

Val Gly His Asp Thr Phe Thr Lys Val Lys Pro Ser Ala Ala Ser
        115                 120                 125

### Alive monomer with His₆ tag

Ala Glu Ala Gly Ile Thr Gly Thr Trp Tyr Asn Gln Leu Gly Ser Thr
1               5                   10                  15

Phe Ile Val Thr Ala Gly Ala Asp Gly Ala Leu Thr Gly Thr Tyr Glu
            20                  25                  30

Ser Ala Val Gly Asn Ala Glu Ser Arg Tyr Val Leu Thr Gly Arg Tyr
        35                  40                  45

Asp Ser Ala Pro Ala Thr Asp Gly Ser Gly Thr Ala Leu Gly Trp Thr
    50                  55                  60

Val Ala Trp Lys Asn Asn Tyr Arg Asn Ala His Ser Ala Thr Thr Trp
65                  70                  75                  80

Ser Gly Gln Tyr Val Gly Gly Ala Glu Ala Arg Ile Asn Thr Gln Trp
                85                  90                  95

Leu Leu Thr Ser Gly Thr Thr Glu Ala Asn Ala Trp Lys Ser Thr Leu
            100                 105                 110

Val Gly His Asp Thr Phe Thr Lys Val Lys Pro Ser Ala Ala Ser His
        115                 120                 125

His His His His His
    130

A monovalent streptavidin (mSA) tetramer includes one wild-type streptavidin monomer subunit that maintains wild-type streptavidin binding affinity for biotin or fragments thereof, and three modified streptavidin subunits that have amino acid sequences that are modified from the wild-type streptavidin amino acid sequence. One type of modified streptavidin subunit used in a monovalent streptavidin tetramer of the invention is referred to herein as a Dead (D) type streptavidin monomer subunit and is a modified streptavidin monomer subunit. A preferred monovalent streptavidin tetramer of the invention includes wild-type and modified streptavidin subunits in a 1:3 ratio. A monovalent streptavidin tetramer contains only a single functional biotin binding subunit. In some preferred monovalent streptavidin tetramers, all three modified streptavidin monomer subunits have the same type of sequence modification. The wild-type streptavidin monomer subunit may also include a purification tag that may be used for the preparation and purification of monovalent streptavidin tetramers.

The wild-type streptavidin monomer subunit may, but need not, include a purification tag that may be used for the preparation and purification of monovalent streptavidin polymers. An example of one alternative method of purifying a monovalent streptavidin polymer without use of a purification tag includes separation of various tetramers with an iminobiotin column. With an iminobiotin column, D4 tetramers would not bind the column and other streptavidin tetramers would be eluted in the order A1D3, A2D2, A3D1, and then A4 with the later tetramers eluting with decreasing pH. Those of ordinary skill in the art will recognize that additional methods can also be used for separating and/or purifying streptavidin tetramers that have differing ratios of A to D streptavidin subunits.

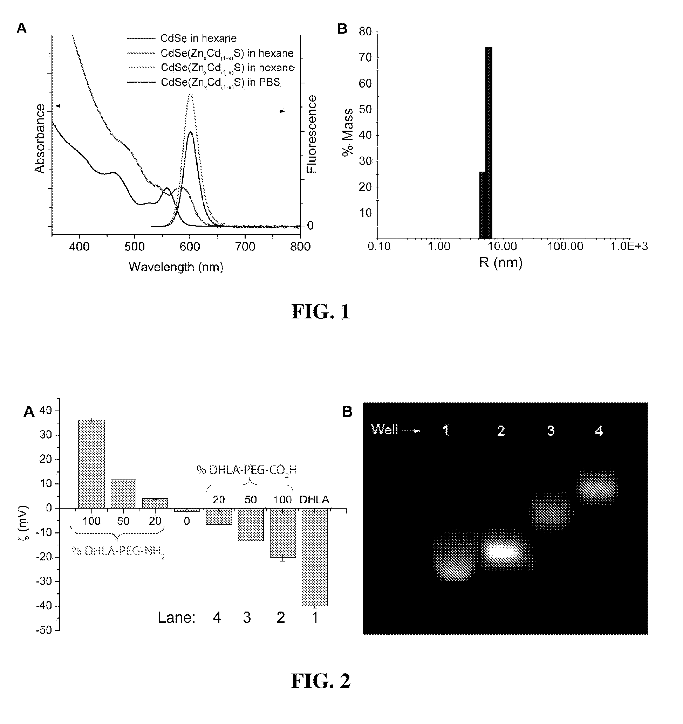

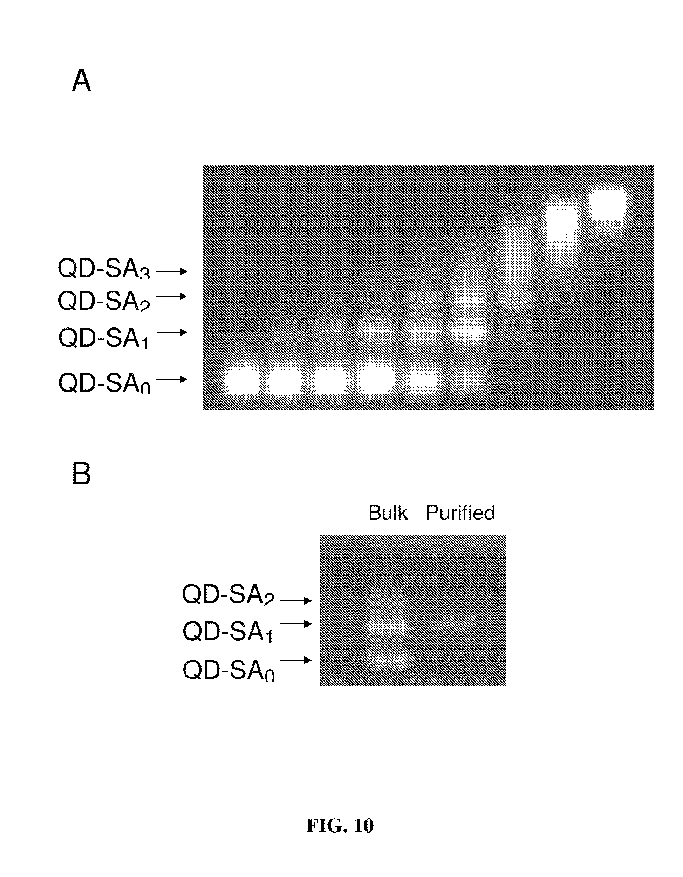

Nanocrystals coated with DHLA-PEG-CO₂H have a negative surface charge and move rapidly upon electrophoresis in an agarose gel, with mobility similar to 1 kilobase DNA (FIG. 2B). His₆-tagged proteins can be efficiently conjugated to these nanocrystals by multidentate interaction of the imidazole groups with the Cd/ZnS shell of the nanocrystal (Clapp,A.R., et al. (2004). J.Am.Chem.Soc. 126, 301-310). When DHLA-PEG-CO₂H nanocrystals were incubated with varying concentrations of monovalent streptavidin, which contains a single His₆-tag, and analyzed by electrophoresis, a striking ladder of nanocrystal mobility was obtained. The discrete and sharp bands observed correspond to different numbers of streptavidin conjugated to the nanocrystal (FIG. 10A). Such a ladder has previously been observed after protein conjugation to nanocrystals, but no purification was shown. See, e.g., Pinaud, F., et al. (2004). J.Am.Chem.Soc. 126, 6115-6123; and Pons, T., et al. (2006). J.Phys.Chem.B Condens.Matter Mater.Surf.Interfaces.Biophys. 110, 20308-20316. Electrophoretic separation allowed easy calibration of the optimal streptavidin concentration to generate nanocrystals bearing principally a single monovalent streptavidin (mSA) per nanocrystal (i.e., monovalent nanocrystals). Conjugation of the mSA was almost quantitative, unlike the <10% yields typically obtained for EDC conjugation of proteins without His-tags to these nanocrystals. Furthermore, the monovalent nanocrystals could be simply purified from the gel by excising the band, which was visible by eye, and separating the nanocrystals from the agarose by centrifugation. After recovery of these monovalent nanocrystals, nanocrystals bound to 0 or 2 copies of streptavidin were not detected when re-examined on an agarose gel (FIG. 10B). This indicated that monovalent nanocrystals were isolated with good purity.

### Examples

**Materials and analysis.** All chemicals were obtained from Sigma Aldrich and used as received. Air sensitive materials were handled in an Omni-Lab VAC glove box under dry nitrogen atmosphere with oxygen levels < 0.2 ppm. All solvents were spectrophotometric grade and purchased from EMD Biosciences. Amine-bearing compounds were visualized on thin layer chromatography (TLC) plates using a ninhydrin solution. All other TLC plates were visualized by iodine staining. NMR spectra were recorded on a Bruker DRX 401 NMR Spectrometer. ESI-MS was measured on an Applied Biosystems QTrap mass spectrometer (Forster City, CA). Samples were dissolved in a solution of actonitrile, water, and acetic acid (50:50:0.01 v/v) at a concentration of 2.5 pmol/µL and introduced via syringe pump at a flow rate of 20 µL/min. All poly(ethylene) glycol compounds produced a distribution of molecular weights, separated by 44 m/z. The mass at the maximum of the distribution is reported in the ESI-MS characterization for all samples. UV-Vis absorbance spectra were taken using an HP 8453 diode array spectrophotometer. Photoluminescence spectra were recorded with a SPEX FluoroMax-3 spectrofluorimeter. The absorbance of all solutions was kept below 0.1 OD to avoid inner-filter effects. Zeta potential measurements were conducted on a Zeta PALS instrument (Brookhaven Instruments Corp).

**Gel Filtration Apparatus.** GFC was performed using an ÄKTAprime Plus chromatography system from Amersham Biosciences equipped with a Superose 6 10/300 GL column. Phosphate buffered saline solution (1X PBS) was used as the mobile phase with a flow rate of 0.5 mL/min. Typical injection volumes were 50 µL. Detection was achieved by measuring the absorption at 280 nm, and the fluorescence spectrum at set time intervals was simultaneously recorded using an Ocean Optics SD2000 fiber optic spectrometer with excitation at 460 nm from an Ocean Optics LS-450 LED light source. The column was calibrated using gel filtration protein standards from Bio-Rad (cat. 151-1901) ranging in MW from 1.3-158 kDa.

**Dynamic Light Scattering.** Light scattering analysis was performed using a DynaPro Dynamic Light Scatterer. All nanocrystal samples were between 0.5-2 µM in concentration and filtered through a 0.02 µm filter before analysis. Typical count rates were between 85-150 kHz. Each autocorrelation function (ACF) was acquired for 10 seconds, and averaged for 10 minutes per measurement. A software filter was employed to discard all ACF fits with sum of squares errors > 15. The resulting ACF was fitted using the Dynamics V6 software employing a non-negative least squares fitting algorithm. Hydrodynamic size data were obtained from a mass weighted size distribution analysis and reported as the mean of triplicate measurements.

**Water solubilization of CdSe(ZnCdS) Core(Shell) nanocrystals.** Exchange of the native TOPO/TOP surface ligands on nanocrystals for the PEG derivatized ligand was carried out according to previously reported procedures, with several modifications (Uyeda, H. T.; et al., J. Am. Chem. Soc. 2005, 127, 3870-3878). To 0.2 mL of nanocrystals in growth solution was added acetone to the point of turbidity. After centrifugation and decantation, 50 µL of neat DHLA-PEG derivatized ligand and 10 µL of MeOH were added. The mixture was stirred at 60°C for 2.5 hr and precipitated by adding 0.3 mL ethanol, 0.05 mL chloroform, and 0.5 mL hexane in succession. Centrifugation at 3000 g for 2 min yielded a clear supernatant, which was discarded. The pellet was dispersed in 0.5 mL of PBS and filtered through a 0.2 µm filter. The sample was purified using gel filtration chromatography in order to remove aggregated nanocrystals, and the fractions were concentrated at 3500 g using a Vivaspin-6 10,000 MWCO spin concentrator. Typical concentrations of nanocrystals preparations were 8 µM. The QY in water was ~40%.

**Quantum yield measurement.** The QY of 565 nm emitting nanocrystals was measured relative to Rhodamine 590 with excitation at 490 nm. Solutions of nanocrystals in PBS and dye in ethanol were optically matched at the excitation wavelength. Fluorescence spectra of QD and dye were taken under identical spectrometer conditions in triplicate and averaged. The optical density at the peak was kept below 0.1, and the integrated intensities of the emission spectra, corrected for differences in index of refraction and concentration, were used to calculate the quantum yields using the expression QY_{NC} = (Absorbance)_{dye}/(Absorbance)_{NC} x (Peak Area)_{NC} /(Peak Area)Dye x (n_{NC solvent})²/(n_{Dye solvent})² x QY_{Dye} (see, e.g., Eaton, D., IUPAC. 1988, 60, 1107-1114.).

**Diamino-PEG (Compound 1).** The synthesis of ligands is also illustrated in Scheme 1 below. Neat poly(ethylene glycol) (Avg MW 400) (20.0 g, 48.3 mmol) was degassed at 80 °C for 1 hr with stirring to remove traces of water. The flask was backfilled with N₂ and cooled on an ice bath before thionyl chloride (10.51 mL, 145.0 mmol) was slowly added. The solution was warmed to RT and stirred for 2 hr. The conversion was monitored by the disappearance of the broad O-H stretch at 3500 cm⁻¹ and the appearance of a C-Cl stretch at 730 cm⁻¹ in the IR spectrum. The product was diluted with DMF (20 mL) and the solvent removed under reduced pressure. This was repeated 3 times to remove all residual traces of thionyl chloride. The sample was dissolved in a solution of sodium azide (9.42 g, 145.0 mmol) in 250 mL DMF and stirred overnight at 85 °C. The solvent was removed under reduced pressure and 200 mL of dichloromethane was added. The precipitate was removed by vacuum filtration and the solvent evaporated under reduced pressure to yield the intermediate azide-functionalized product. The conversion was confirmed by the appearance of a sharp azide stretch at 2100 cm⁻¹ and the disappearance of the C-Cl stretch at 730 cm⁻¹ in the IR spectrum. The sample was dissolved in 300 mL of tetrahydrofuran, and triphenylphosphine (27.9 g, 106 mmol) was added. The solution was stirred at RT for 4 hr before adding 4 mL of water and stirring overnight. The solvent was removed *in vacuo* and 100 mL of DI water was added. The precipitate was removed by vacuum filtration and the filtrate washed with toluene (3 x 50 mL). The solvent was removed *in vacuo* to yield the pure product as light yellow oil (15.5 g, 78%). ESI-MS: *m*/*z* 457 [M + H]⁺. ¹H NMR (400 MHz, CDCl₃): δ (ppm) 3.53 (m, 28 H), 3.39 (t, *J* = 5.2 Hz, 4H), 2.74 (t, J = 5.2 Hz, 4H), 1.27 (s, 4H).

**Thioctic Acid NHS Ester (TA-NHS).** To a solution of lipoic acid (5.00 g, 24.23 mmol) and NHS (3.35 g, 29.1 mmol) in 150 mL THF at 0 °C was added slowly a solution of DCC (6.00 g, 29.1 mmol) in 10 mL THF. The mixture was warmed to room temperature and stirred for 5 hr. The precipitate was removed by vaccum filtration and the solvent evaporated in vacuo. The crude product was redissolved in 100 mL of ethyl acetate and filtered once more by vacuum filtration. The product was recrystallized from a solution of hot ethyl acetate:hexane (1:1 v/v) as a pale yellow solid (5.88 g, 80%). 1H NMR (400 MHz, CDCl3): δ (ppm) 3.58 (m, 1H), 3.13 (m, 2H), 2.84 (s, 4H), 2.63 (t, J = 7.1 Hz, 2H)), 2.50 (m, 1H), 1.99-1.46 (m, 7H).

**TA-PEG-NH₂ (Compound 2).** To a solution of compound **1** (12 g, 29.1 mmol) and sodium bicarbonate (2.44 g, 29.1 mmol) in DMF/water (100 mL, 50:50 v/v) at 0 °C was added dropwise a solution of lipoate-NHS (1.60 g, 5.27 mmol) in 10 mL DMF over 1 hr. The solution was warmed to RT, stirred overnight, and extracted with chloroform (3 x 30 mL). The combined organic extracts were washed with water (3 x 30 mL), dried over Na₂SO₄, filtered, and the solvent evaporated. The crude product was purified by alumina column (dichloromethane/methanol 95:5) to give the final product as a yellow oil (1.90 g, 60%). ESI-MS: *m*/*z* 645 [M + H]⁺. ¹H NMR (400 MHz, CDCl₃): δ (ppm) 3.63 (m, 26H), 3.52 (t, J = 5.2 Hz, 2H), 3.47 (t, *J* = 5.2 Hz, 2H) 3.10 (m, 2H), 2.86 (t, *J* = 5.2 Hz, 2H), 2.40 (m, 1H), 2.17 (t, *J* = 6.5 Hz, 2H), 1.99 - 1.46 (m, 7H).

**TA**-**PEG**-**CO₂H (Compound 5).** To a solution of compound **2** (1.90 g, 3.16 mmol) and triethylamine (0.320 g, 3.16 mmol) in dichloromethane (30 mL) was dripped slowly a solution of methylmalonylchloride (0.475 g, 3.48 mmol) in dichloromethane (10 mL) at 0 °C. The solution was stirred at RT for 4 hr and the solvent removed *in vacuo*. The crude product was purified by silica column (dichloromethane/methanol 95:5) and the solvent evaporated to give the pure product (compound **3**) as a yellow oil (1.97 g, 89%). Methylester deprotection was achieved by stirring with 3.5 equiv of NaOH in methanol for 5 hr at 60 °C. The solvent was removed *in vacuuo* after neutralizing to pH 7 with 3M HCl. The product was dissolved in water, acidified to pH 2, and extracted with chloroform (3 x 20 mL) to yield the pure product in quantitative yield. ESI-MS: *m*/*z* 731 [M - H]⁻. ¹H NMR (400 MHz, CDCl₃): δ (ppm) 3.70-3.52 (m, 36 H), 3.51-3.35 (m, 6H), 3.14 (m, 2H), 2.45 (m, 1H), 2.20 (t, *J* = 7.3 Hz, 2H), 1.96-1.36 (m, 7H).

**General procedure for disulfide ring opening (Compounds 3 and 6).** To a solution of TA-PEG in 0.25 M NaHCO₃ (20 mL) at 0 °C was slowly added 4 equivalents of sodium borohydride over a 30 min period. The solution was stirred for 2 hr on ice, acidified to pH 2 with 3 M HCl, and extracted with chloroform (3 x 15 mL). The combined organics were dried over MgSO₄ and filtered. The solvent was removed *in vacuo* to yield the product as a colorless oil.

**Agarose gel electrophoresis.** Electrophoresis of nanocrystals was performed using a Minicell Primo (Thermo) with 1% Omnipur agarose (EMD) in TAE (40 mM Tris-acetate, 1 mM EDTA, pH 8.3) at 7.9 V/cm for 15 min. Nanocrystals were diluted to 150 nM in TAE and mixed with 6x loading buffer (16% sucrose) before loading onto the gel. Gels were visualized under 305 nm UV with a ChemiImager 5500 (Alpha Innotech Corporation). Monovalent streptavidin in PBS was prepared as previously described (Howarth et al. 2006). Wild-type streptavidin was dissolved at 1 mg/mL in PBS. To test the effect of the His₆-tag on nanocrystal conjugation, 5 µL of 3 µM nanocrystals emitting at 600 nm and coated with DHLA-PEG-CO₂H in 10 mM sodium borate pH 7.4 were incubated with 5 µL 27 uM monovalent streptavidin in PBS for 1 hr at 24 °C. Streptavidin conjugation was then tested by electrophoresis. These nanocrystals were used at 50 nM for labeling of cells.

**Purification of monovalent nanocrystals.** 0.5 µL nanocrystals at 8 µM in PBS were incubated with the indicated volumes of monovalent streptavidin (19 µM) or scFv (13 µM) in PBS in a total volume of 5 µL for 1 hr at 24 °C. Monovalent streptavidin was prepared as described (Howarth et al. 2006). mSA and scFv each contained a single His₆-tag that stably binds to the Cd/ZnS shell (Clapp *et al*., 2004). Addition of 1-ethyl-3-diisopropylaminocarbodiimide (EDC) did not change the conjugation efficiency. Analysis of nanocrystal-protein conjugation was performed by electrophoresis using a Minicell Primo (Thermo) with 1% Omnipur agarose (EMD) in 10 mM sodium borate (adjusted to pH 8.0 using 1 M HCl) at 7.9 V/cm for 15 min. 6x loading buffer (16% sucrose in ddH₂O) was added to samples before loading. For purification, buffer was cooled on ice, the electrophoresis apparatus was surrounded in ice and the gel was run at 6.4 V/cm for 20 min. A molar ratio of 1 nanocrystal to 1 mSA was used to generate the samples for monovalent nanocrystal purification. Gels were visualized under 305 nm UV with a ChemiImager 5500 (Alpha Innotech Corporation) for analysis, or with the naked eye under ambient light for purification. Bands of interest were excised with a scalpel, placed in a Nanosep MF 0.2 µm column (Pall), and centrifuged at 5,000 g for 3 minutes at 24 °C. This centrifugation spun the buffer and nanocrystal-protein conjugates out of the agarose into the collecting tube below (see, e.g., Loweth, C.J., et al. (1999). Angewandte Chemie-International Edition 38, 1808-1812).

Extraction efficiency from the agarose was 30-50% (data not shown). **Conjugation of monovalent streptavidin dye-functionalized nanocrystals.** To conjugate streptavidin to the nanocrystals, 5 µL of 3 µM control or dye-conjugated nanocrystals in 10 mM sodium borate pH 7.4 were incubated with 5 µL 27 uM monovalent streptavidin in PBS for 1 hr at 24 °C. These nanocrystals were then used at 50 nM for labeling of cells.

**Cell culture, labeling and imaging.** HeLa (human carcinoma) and COS7 (African Green Monkey Kidney) cells were grown in DMEM with 10% Fetal Calf Serum, 50 U/mL penicillin and 50 µg/mL streptomycin. Transfections were performed with Lipofectamine 2000 (Invitrogen) according to manufacturer's instructions and cells were imaged the day after transfection.pEYFP-H2B (Platani Swedlow 2002) was a kind gift of A.K. Leung (MIT). This encodes histone H2B fused to EYFP, as a nuclear-localized co-transfection marker. LDLR-AP had AP inserted after the signal sequence of human low density lipoprotein receptor, allowing extracellular display. LDLR in pEGFP (Clontech) was a kind gift of T. Kirchhausen (Center for Blood Research, Harvard Medical School). The cytoplasmic co-transfection marker BFP was a gift from R. Y. Tsien (UCSD). The human EGFR gene in pcDNA3 (Invitrogen) was a gift from K. D. Wittrup (MIT). AP was inserted in pEGFP-LDLR by inverse PCR with the primers 5'-cttcgaggcccagaagatcgagtggcacgagactgtgagcaagggcgaggag and 5'-atatcgttcaggccacttctgtcgccaactgcag. EGFP was removed by QuikChange™ with the primer 5'-cccagaagatcgagtggcacgaggggggagaattcgacagatgtg and its reverse complement. The construct was verified by sequencing.

CHO ldlA7 (A7) are a variant of CHO lacking endogenous LDLR and were a kind gift from M. Krieger (MTT, US). CHO, HeLa and A7 were grown in DMEM with 10% Fetal Calf Serum, 50 U/mL penicillin, 50 µg/mL streptomycin, 1 mM pyruvate and L-proline at 69 mg/L. DMEM is reported to contain no biotin (Invitrogen) and 100% Fetal Calf Serum contains - 90 nM biotin (see, for example, Baumgartner, M.R., et al. (2004). Am.J.Hum.Genet. 75, 790-800). Cell-lines were transfected using 1 µl Lipofectamine 2000 (Invitrogen), 0.1 µg of the gene of interest and 5 ng co-transfection marker per well of a 48-well plate. Where cells were cotransfected with BirA-ER and an AP fusion, 0.1 µg of each plasmid was used per well. Cells were imaged the day after transfection. Primary hippocampal cultures were prepared from E18-19 rats, in accordance with MIT guidelines. Briefly, hippocampi were dissociated with papain and neurons were plated onto 12 mm coverslips in Basal Medium Eagle (BME) plus 5% Fetal Calf Serum at a density of 200,000 cells/well. The coverslips were pre-coated with poly-D-lysine (Mw 300,000) (Sigma) (0.1 mg/ml) and mouse laminin (Invitrogen) (5 µg/ml) overnight After cultures were grown at 37 °C for 8 h, the medium was replaced with Neurobasal medium (Invitrogen) supplemented with 2% B27 (Invitrogen), 0.5 mM glutamine, 25 U/mL penicillin and 25 µg/mL streptomycin for further culture. Neurons were transfected using calcium phosphate at DIV6.

**Plasmids.** pEYFP-H2B was a kind gift of A.K. Leung (MIT, US). This encodes human histone H2B fused to EYFP, as a nuclear-localized co-transfection marker. Enhanced Blue Fluorescent Protein in pcDNA3 (a kind gift of R.Y. Tsien) was used as a cytosolic co-transfection marker. LDLR-AP had AP and inserted after the signal sequence of human LDLR, so that the AP was exposed at the cell surface. LDLR in pEGFP (Clontech) was a kind gift of T. Kirchhausen (Harvard Medical School, US). AP was inserted in pEGFP-LDLR by inverse PCR with the primers 5' cttcgaggcccagaagatcgagtggcacgagactgtgagcaagggcgaggag and 5' atatcgttcaggccacttctgtcgccaactgcag. EGFP was removed by QuikChange™ (Stratagene) with the primer 5' cccagaagatcgagtggcacgaggggggagaattcgacagatgtg and its reverse complement LDLR-Ala was generated from LDLR-AP by Quikchange™ using the primers previously described (see Chen, L, et al. (2005). Nat.Methods 2, 99-104; and Chen, L, Ting, A.Y. (2005). Curr.Opin.Biotechnol. 16, 35-40). These changes were verified by DNA sequencing. FH LDLR-AP contained the mutation and was generated by Quikchange™ with the primer 5' CCTTCTATGGAAGAACTGACGGCTTAAGAACATCAAC and its reverse complement GluR2-AP in CMVbipep-neo has been described (Howarth *et al*., 2005). The post-synaptic marker Homer1b-GFP in pCI was a kind gift of Yasunori Hayashi (MIT, US). BirA-YFP-ER has the structure: Ig signal sequence-HA tag-BirA-YFP-KDEL and was generated by PCR to insert *E*. *coli* BirA and yellow fluorescent protein (YFP) into pDisplay (Invitrogen) and QuikChange™ to insert the KDEL ER retention sequence and a stop codon directly after BirA.

YFP was deleted to give BirA-ER by Quikchange™. EphA3-AP in pEF-BOS was a kind gift from Martin Lackmann (Monash University, Australia). AP was inserted by inverse PCR immediately after the signal sequence of human EphA3, using the primers
5'GTTCTCGACAGCTTCGGGGGCCTGAACGATATCTTCGAGGCCCAGAAGATCGAGT GGCACGAGGAACTGATTCCGCAGCC and
5'GGCTGCGGAATCAGTTCCTCGTGCCACTCGATCTTCTGGGCCTCGAAGATATCGTT CAGGCCCCCGAAGCTGTCGAGAAC. Human carcinoembryonic antigen (CEA) in pCI was a kind gift of G. Prud'homme (University of Toronto, Canada). Human EGFR-AP in pcDNA3 and AP-CFP-TM in pDisplay have been described (Chen *et al*., 2005).

**Non-specific binding of nanocrystals.** HeLa, cooled to 4 °C in PBS for 5 min to minimize endocytosis, were incubated with 40 nM nanocrystals in PBS with 0.5% dialyzed bovine N,N-dimethyl casein (Calbiochem) for 10 min at 4 °C. Cells were then washed 4x with ice-cold PBS and imaged in PBS.

**Biotinylation and labeling of cells.** Cells were biotinylated in PBS 5mM MgCl₂ with 2.6 µM biotin ligase and 10 µM biotin-AMP at 24 °C for 10 min (Howarth *et al*., 2006). Cells were then washed 4x with PBS and incubated for 5 min at 24°C with 20 nM nanocrystals in PBS with 0.5% dialyzed bovine N,N-dimethyl casein (Calbiochem). Cells were washed 3x in PBS and imaged in PBS at 24°C.

For imaging of cell-lines expressing BirA-ER, cells were incubated in normal growth medium supplemented with 10 µM biotin (Tanabe USA) from 4 hours after transfection. The next days cells were washed 4x in PBS and incubated for 5 min at 24 °C with 20 nM monovalent nanocrystals in PBS with 0.5% dialyzed casein. For specificity testing, cells were incubated with monovalent streptavidin Alexa Fluor 568 (prepared as described (Howarth *et al*., 2006))at 100 nM in PBS with 3% dialyzed Bovine Serum Albumin for 10 min at 4 °C. Cells were then washed 3x in PBS and imaged live. For analysis of EphA3-AP clustering, cells were biotinylated as above for 10 min, washed 3x in PBS and then incubated with 10 nM monovalent nanocrystals or multivalent nanocrystals (molar ratio of 6 mSA:nanocrystal) for 14 min at 37 °C. Cells were washed 3x in ice-cold PBS and imaged at 4 °C. For biotinylation and imaging of neurons, PBS was replaced with Tyrode's buffer.

**EGF Receptor Labeling with Streptavidin Linked to nanocrystals by EDC Coupling.** COS7 cells were transfected with 0.2 µg pcDNA3 EGFR and 7.5 ng pcDNA3 BFP per well of a 48-well plate. The next day cells were incubated in PBS with 5 mM MgCl₂, 0.5% dialyzed casein and 90 nM biotinylated EGF (biotin-XX-EGF from Invitrogen: human EGF conjugated at a single site to biotin via a long spacer arm) for 5 min at RT. Cells were washed four times with PBS and incubated with PBS, 0.5% dialyzed casein and 70 nM 20% aminoNC605-wtSA for 5 min at RT, before washing four times in PBS and imaging in PBS. As a negative control, NC605-wtSA was incubated with a 500-fold excess of free biotin (Tanabe, USA) for 5 min at RT, before adding to cells. For video imaging, COS7 cells were labeled as above but with 20 nM 20% aminoNC605-wtSA and were maintained in the microscope at 37 °C using an environmental control system (Solent Scientific).

**Imaging of FRET between Nanocrystals and Dye while Bound to EGF Receptor.** A five-fold molar excess of hSA in PBS was incubated with 20% aminoNC558 or 20% aminoNC558-Alexa Fluor 568 for 30 min at RT, allowing stable binding of the His₆-tag of hSA to the nanocrystal shell. HeLa were transfected with 0.2 µg pcDNA3 EGFR and 5 ng H2B-YFP per well of a 48-well plate. The next day, cells were incubated in PBS with 5 mM MgCl₂, 0.5% dialyzed casein and 60 nM biotinylated EGF (biotin-XX-EGF from Invitrogen: human EGF conjugated at a single site to biotin via a long spacer arm) for 5 min at 4 °C, to stop receptor internalization. Cells were washed four times with cold PBS and incubated with PBS, 0.5% dialyzed casein and 40 nM QD558-hSA or QD558-hSA-Alexa Fluor 568 for 5 min at 4 °C, before washing four times in cold PBS and imaging.

**Fluorescence and phase contrast microscopy.** Images of HeLa cells were collected on live cells using a Zeiss Axiovert 200M inverted epifluorescence microscope with a 40x oil-immersion lens and a Cascade II camera (Photometrics) with intensification set at 3500. YFP (495DF10 excitation, 515DRLP dichroic, 530DF30 emission), Alexa Fluor 568 (560DF20 excitation, 585DRLP dichroic, 605DF30 emission), QD 565 (405DF20 excitation, 515DRLP dichroic, 565DF20 emission) and QD 605 (405DF20 excitation, 585DRLP dichroic, 605DF30 emission) images were collected and analyzed using Slidebook software (Intelligent Imaging Innovations). Typical exposure times were 0.1-0.5 s. AlexaFluor 568 fluorescence was bleached by 30 s of intense 405DF20 excitation. Fluorescence images were background-corrected.

**Atomic Force Microscopy.** Biotinylated DNA was generated by PCR from pIVEX-BirA (a kind gift from A. Griffiths, MRC Laboratory for Molecular Biology, UK) using Taq polymerase with the primers 5' GTCGCCATGATCGCGTAGT and 5' biotin-triethylene glycol-GCGTTGATGCAATTTCT (Eurogentec) using the conditions 95 °C 30 s, 50 °C 30 s, 72 °C 1 min for 27 cycles. The DNA was run on an ethidium bromide-stained 2% agarose gel in TAE at 9.3 V/cm for 30 min. The unique 119 bp PCR product was visualized under UV, extracted into ddH₂O using a QIAquick gel extraction kit (Qiagen), and aliquots stored at -20 °C. 9 nM Biotinylated DNA was incubated with 3 nM nanocrystals for 1 h at 24 °C in 10 mM sodium borate pH 7.3. 20 µL 0.1 % poly-L-lysine (Mw 500-2000) (Sigma) was pipetted on freshly cleaved muskovite mica V2 (Electron Microscopy Sciences). After incubation for 3 min, the mica was washed with 1mL of ddH₂O and dried in a nitrogen stream. 20 µL DNA-nanocrystal complex was pipetted onto the mica. After 6 min, the mica was rinsed with 1 mL of ddH₂O and dried in a nitrogen stream. The mica was imaged using a Dimension 3100 atomic force microscope (Digital Instruments, Santa Barbara CA) in tapping mode, using an etched TESP silicon probe with a nominal tip radius of <10 nm (Veeco Probes) operating at a resonant frequency of 273.3 kHz. Height and phase images were collected simultaneously across a 1 x 1 µm area, at a scan rate of 2 Hz and a resolution of 256 x 256 pixels. The resulting images were analyzed using Nanoscope v 5.30 software (Digital Instruments).

**Fluorescence Correlation Spectroscopy.** Two photon Fluorescence Correlation Spectroscopy measures the autocorrelation of the emission of nanocrystals in solution under a microscope objective, to determine the dynamics of time spent in a calibrated focal volume and therefore the diffusion constant. Diffusion constant is then related to hydrodynamic diameter via the Stokes-Einstein relation. The homebuilt instrument begins with 780nm light from a Coherent Ti-Sapphire oscillator pumped with an Ar+ Ion laser (Coherent Mira) and operating with a pulse-width of -120 fs and a repetition rate of 80 MHz. The beam is sent through a 40x, 1.2 NA water immersion objective (Zeiss). Fluorescence is collected through the same objective, filtered through a 720 longpass dichroic (Chroma), 400nm holographic notch filter and an appropriate bandpass. Then, the light passes through a 50/50 beam splitter to two avalanche photodiodes (EG&G and SPCM-AQR14). The signal is cross-correlated by a multi-tau autocorrelator card (ALV GmbH). This cross correlation reduces the noise of the measurement and eliminates artifacts from the after-pulsing of the photodiodes. The 3D focal volume waist and aspect ratio is calibrated according to a standard 3D Gaussian approximation. The aspect ratio is first calculated using a known concentration of R_{H} = 22 nm beads (Duke Scientific, product G40). This ratio is then applied to a series of freshly filteted beads (R_{H}=22 nm, 28.5 nm, 35.5 nm, 44 nm, Duke Scientific, G40, G50, G75, G80) to determine the beam waist. R² values for these fits were greater than 0.999. Samples were measured with a range of excitation powers, to ensure that we were not in a regime where blinking, saturation, and photobleaching would influence the measurements (see, e.g., Larson, D. R., et al. Science 300[5624], 1434-1436. 2003). For the final measurement, DHLA-PEG-CO₂H nanocrystals were excited with 0.75 mW on the back focal plane of a lightly overfilled objective and the commercial nanocrystal-SA were excited with 0.5 mW. Samples were measured for 10 runs of 30 s each and the r² values were >0.998 for the average curve. Hydrodynamic diameter was calculated as described (see, e.g., Schwille, P., et al. (1996). Biochemistry 35, 10182-10193).

**Antibody Production.** Secretion vectors for the anti-CEA scFv sm3E have been described (Graff, C.P., et al. (2004). Protein Eng Des Sel 17, 293-304). To prevent protein dimerization, a disulfide bond was inserted between the V_{H} and V_{L} domains of the scFv by mutating residues R44 and G234 to cysteine using QuikChange™. QuikChange™ was also used to insert a cysteine at the C-terminus of the scFv, directly preceding the His₆ tag, for PEGylation. Plasmids were transformed into YVH10 yeast using the EZ Yeast Kit (Zymo Research) and plated on SD-CAA media supplemented with 40 µg/mL tryptophan. Individual colonies were grown in 1 L flasks and secretion induced for 48 h at 37 °C as described (Graff *et al*., 2004). The cleared supernatant was concentrated using a 10 kDa ultrafiltration membrane (Millipore) and the His-tagged protein purified with Talon metal affinity resin (BD Biosciences) following the manufacturer's batch-column protocol. Monomeric scFvs were further purified by size exclusion chromatography on a Superdex 75 column (GE Healthcare) and eluted into PEGylation buffer (100 mM Na₂HPO₄, 500 mM NaCl, 2 mM EDTA, pH 6.5). Unmodified scFv is too small (27 kDa) to give discrete bands after conjugation to nanocrystals, but PEGylation of the scFv significantly increases the effective hydrodynamic size, without impairing antigen recognition. For PEGylation, scFvs at a concentration of 0.5-1 mg/mL were co-incubated with a 5 fold molar excess of 5 kDa PEG-maleimide (Nektar) and immobilized Tris[2-carboxyethyl]phosphine hydrochloride (TCEP) reducing gel (Pierce) at a concentration of 150 µL gel per 1 mL reaction. Using TCEP resin instead of soluble TCEP or dithiothreitol to reduce the C-terminal cysteine prevented reduction of the partially buried disulfide bonds in the protein. The reaction mixture was incubated at 25°C for 5 h on a rocker. The TCEP resin was then removed by centrifugation. Unreacted PEG was removed by ion exchange chromatography on a Hi-Q column (GE Healthcare) equilibrated with 20 mM Tris HCl, pH 8.2. PEGylated scFvs were separated from unconjugated scFvs on a Superdex 75 column at a flow rate of 0.5 mL/min and eluted in PBS. The PEGylation efficiency and conjugate purity were assessed by SDS-PAGE.

**Ligand Synthesis.** Diamine functionalized polyethylene glycol was synthesized from cheap and commercially available PEG (avg. MW 400) starting material via a simple three step reaction in high yield with minimal purification steps required (Scheme 1). The overall yield was 89% on a 30 g scale. All intermediate products were easily monitored by FTIR spectroscopy.

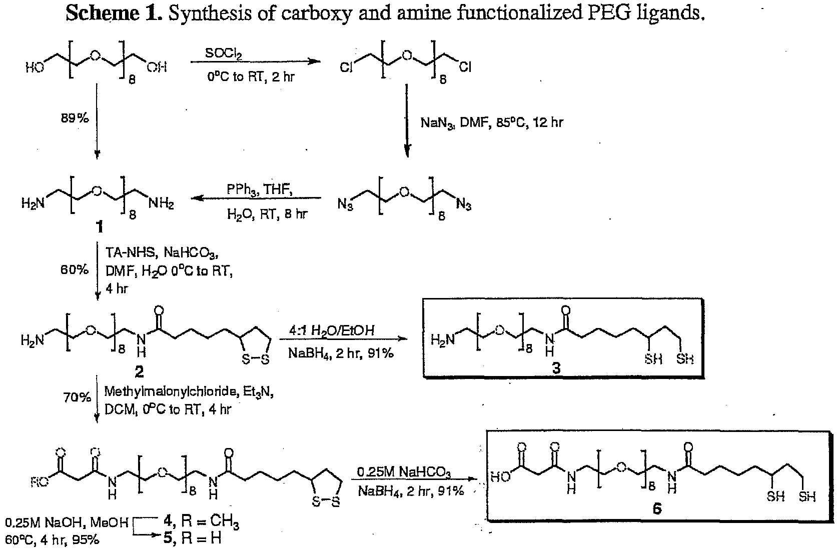

After ligand exchange with compound **3** (DHLA-PEG-NH₂), nanocrystals were water dispersible and bore surface amino groups that were amenable to further covalent modification via simple N-hydroxysuccinimidyl (NHS) coupling chemistry.

Alternatively, compound **2** was modified further to yield compound **6** (DHLA-PEG-CO₂H), which after ligand exchange gave water dispersible nanocrystals bearing carboxyl surface groups.

The synthetic schemes presented provide a simple means toward heterobifunctional ligands, which bear a multidentate coordinating moiety at one terminus for strong coordination to the nanocrystal surface, a short spacer to convey water solubility and to reduce non-specific binding, and an ionizable functionality at the end terminus that enables further derivatization. Moreover, all coupling can be achieved via amide-bond formation, which is more stable towards hydrolysis than ester bonds. This feature can be especially relevant for stability in the context of cell labeling, in which the cellular environment may contain a number of non-specific esterases (see, e.g., Gerolf, V. Z., ACTA Path Micro. IM. B. 1970, 78, 258-260).

**Ligand Exchange and Characterization of Functional Hydrophilic Nanocrystals.** Nanocrystals with emission at 565 and 600 nm were synthesized according to known literature procedures, with several modifications. Briefly, CdSe cores were formed through the rapid injection of Cd and Se precursors into a hot solvent system. The cores were then purified by precipitation, redispersed in a coordinating solvent, and overcoated with approximately five monolayers of an alloy ZnₓCd(₁₋ₓ)S shell, with *x* ≈ 0.7. The formation of the alloy shell was accompanied by a large red-shift of the absorbance maximum by as much as 30 nm upon shell growth, as illustrated in FIG. 1A. FIG. 1A shows NC605 absorption spectra of CdSe cores (black) and CdSe(ZnₓCd₍₁₋ₓ₎S) core(shell) after overcoating (red). Fluroescence spectra with matched excitation at 520 nm of NC605 in hexane after one cycle of precipitation (green, QY=65%), and in PBS buffer after ligand exchange with compound **3** (blue, QY = 43%), showing a minimal decrease in QY.

The use of an alloyed shell was found to enhance the quantum yield of nanocrystals by as much as two fold upon transfer to aqueous solution compared with nanocrystals overcoated with a pure ZnS shell. Ligand exchange of CdSe(ZnₓCd₍₁₋ₓ₎S) core(shell) nanocrystals with DHLA-PEG-NH₂ (compound **3**) and DHLA-PEG- CO₂H (compound **6**) was carried out according to previously reported procedures (e.g., Uyeda, H. T.; et al., J. Am. Chem. Soc. 2005, 127, 3870-3878), with the following modifications. Briefly, 200 µL of nanocrystals in growth solution were precipitated by the addition of a polar solvent to yield -10 mg of dry pellet to which 50 µL of neat DHLA-PEG derivatized ligand and 10 µL of MeOH were added. The mixture was stirred gently under N₂ at 60 °C for 2.5 hours and precipitated by the addition of ethanol, chloroform, and hexane. Centrifugation at 3000 g for 2 min yielded a clear supernatant containing excess ligand and organic soluble impurities, which were discarded. The pellet was dispersed in 0.5 mL phosphate buffered saline (PBS, pH 7.4), filtered through a 0.2 µm filter to remove aggregates, and purified by two cycles of ultrafiltration. Typical QY for nanocrystals capped with DHLA-PEG-NH₂ (compound **3**) was 33%, while QY for nanocrystals capped with DHLA-PEG-CO₂H (compound **6**) was as high as 43% after water solubilization.

The HD of DHLA-PEG-CO₂H capped nanocrystals was measured by dynamic light scattering (DLS) and found to be -11.2 nm for NC605. See FIG. 1B, which shows epresentative DLS data for NC605 ligand exchanged with compound **3** in PBS, with HD = 11.2 nm. Not surprisingly, the HD of the same nanocrystals ligand exchanged with compound **3** or compound **6** exhibited nearly identical HD on the order of -11 nm, due to the similar size of the ligand coating.

Stability of nanocrystals ligand exchanged with compound **3** or compound **6** was tested in aqueous buffer ranging from pH 5 to pH 9. After 2 days under ambient laboratory conditions, the nanocrystals remained well-dispersed in solution, highlighting the excellent pH stability of nanocrystals coated with DHLA-PEG based ligands, consistent with previous reports.

**Tuning the surface charge and functional valency using mixed ligand systems.** Nanocrystals ligand exchanged with compound **3** or compound **6** will have surfaces that are positively and negatively charged, respectively, in neutral buffer, which may contribute to non-specific binding despite the passivating PEG spacer. The surface charge, along with the proportion of amine or carboxy functionality on the nanocrystal surface, can be tuned by performing ligand exchange using a mixture of compound **3** or compound **6** with neutral DHLA-PEG₈-OH, synthesized according to previously reported procedures. The concept of ligand exchange using a mixture of different DHLA-based ligands was first demonstrated by Uyeda, H. T.; et al., J. Am. Chem. Soc. 2005, 127, 3870-3878. Similarly, we prepared mixed ligand aqueous nanocrystals by performing ligand exchange using a mixture of DHLA-PEG-NH₂ or DHLA-PEG-CO₂H with DHLA-PEG-OH in varying proportions. The surface charge of the resulting nanocrystals were analyzed by zeta-potential and agarose gel electrophoresis (see FIGS. 2A and 2B). Measurements indicate that purely DHLA-PEG-NH₂ capped nanocrystals have a zeta-potential of +36.2 mV, which decreased in an approximately linear fashion with decreasing -NH₂ composition in the ligand blend. A similar trend was observed for the carboxy-coated nanocrystals (FIG. 2A), a trend which was mirrored in the electrophoretic gel mobility (FIG. 2B). Nanocrystals coated with 100% DHLA were also included in the measurements for comparison, revealing that DHLA capped nanocrystals have a higher negative charge than 100% DHLA-PEG-CO₂H coated nanocrystals, due to the DHLA capped nanocrystals having a higher ligand packing density and/or a smaller hydrodynamic radius.

Ligand exchange using mixed ligand systems tunes the surface charge and controls the per-particle valency of functional groups on the nanocrystal surface. The quantity of reactive functional groups per nanocrystals was examined by exposing amine-coated nanocrystals to fluorescamine, a fluorogenic probe which reacts rapidly with primary amines to form a fluorescent product that can be monitored at 487 nm. Nanocrystals ligand exchanged with various mixtures of -NH₂ and -OH functionalized DHLA-PEG were purified by repeated ultrafiltration to remove excess ligand and reacted with excess fluorescamine. By calibrating the fluorescence intensity of the fluorescamine after reaction with samples of **3** at known concentration, an estimate of ~140 amines/nanocrystal was obtained for 100% DHLA-PEG-NH₂ ligand exchanged nanocrystals, a number consistent with previously reported values obtained using a slightly different method (Ballou, B.; et al., Bioconjugate Chem. 2004, 15, 79-86). Furthermore, an approximately linear correlation was obtained for the amount of amine detected per nanocrystals with the percentage of amine-terminated ligands used in the cap-exchange blend.

These results suggested that the use of a mixed ligand blend of charged and neutral DHLA-PEG based ligands offers a simple and robust way to precisely tune the final surface composition and charge of the resulting water solubilized nanocrystals. Such mixed ligand systems can offer a means towards nanocrystals that present surface amino or carboxyl groups in a high enough per-particle valency for applications, while maintaining a minimally-charged particle surface that can mitigate nonspecific binding.

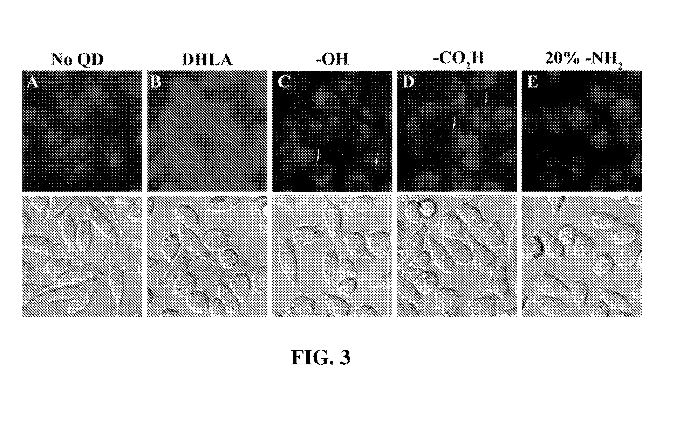

**Assessment of non-specific cell binding.** The issue of non-specific binding must be addressed in order for nanocrystals to be useful for cellular labeling applications, especially for single molecule imaging where a high signal to noise ratio is critical for obtaining meaningful data. Non-specific cell binding in nanocrystals imaging experiments has been is partly attributed to electrostatic interactions of the cell surface with charged nanocrystal ligand coatings, and may also be caused by hydrophobic interactions of lipids in the cellular membrane with residual hydrophobic capping ligands on the nanocrystal surface as a result of incomplete ligand exchange. See, e.g., Bentzen, E. L., et al., Bioconjugate Chem. 2005, 16, 1488-1494. Nanocrystals coated with various ligands were exposed to a human cell line at 4 °C for 10 min. The cells were then washed with buffer to remove unbound nanocrystals, and the residual nanocrystal fluorescence was imaged to investigate the effect of non-specific binding as a function of ligand composition. See FIG. 3.

All Fluorescence images in FIG. 3 were captured under identical camera conditions and displayed at the same contrast level such that they can be directly compared. The control sample (FIG. 3A) showed emission from cell autofluorescence. Nanocrystals coated with negatively charged DHLA showed significant non-specific binding to both cells and glass (FIG. 3B). Neutral DHLA-PEG-OH coated nanocrystals showed minimal non-specific binding, as expected (FIG. 3C). In this case, the cell autofluorescence was clearly seen, and a few individual nanocrystals that bound non-specifically are indicated by arrows. Surprisingly, DHLA-PEG-CO₂H coated nanocrystals, which have a similar charge to that of DHLA coated nanocrystals as indicated by agarose gel shift, also showed minimal non-specific cell binding, comparable to that seen with the neutral PEG-OH functionalized nanocrystals (FIG. 3D). The lack of non-specific binding of DHLA-PEG-CO₂H coated nanocrystals, despite the negative charge, highlights the important role of the PEG spacer in reducing non-specific binding (FIG. 3C). However, the degree of non-specific binding increased when ligand exchange was performed with a DHLA-PEG-CO₂H sample in which the dihydrolipoate moiety had partially oxidized over the course of a few days under ambient conditions to its ring-closed form. This increase in non-specific binding was attributed to a reduction in the efficiency of the ligand exchange, possibly resulting in incomplete displacement of the initial hydrophobic capping shell. For minimal non-specific binding, it was important to store DHLA-based compounds under N₂ atmosphere in the dark at < 4 °C to minimize oxidation.

On the other hand, DHLA-PEG-NH₂ coated nanocrystals exhibited severe non-specific binding to the cell membrane (data not shown). This was attributed to electrostatic interactions of the highly positively charged nanocrystal with the negatively charged cell membrane. However, nanocrystals coated with a 20% mixture of -NH₂ to -OH functionalized DHLA-PEG (FIG. 3E) did not show significantly more non-specific binding than -OH functionalized DHLA-PEG nanocrystals alone.

These results indicate that both DHLA-PEG-CO₂H and 20% DHLA-PEG-NH₂ coated nanocrystals exhibit minimal non-specific binding to cells, making them suitable for cell labeling and single particle imaging applications. The use of 20% AHLA-PEG-NH₂ coated nanocrystals also demonstrate the importance and versatility of performing ligand exchange using mixed ligand systems for tailoring the surface properties of the nanocrystal for the desired application.

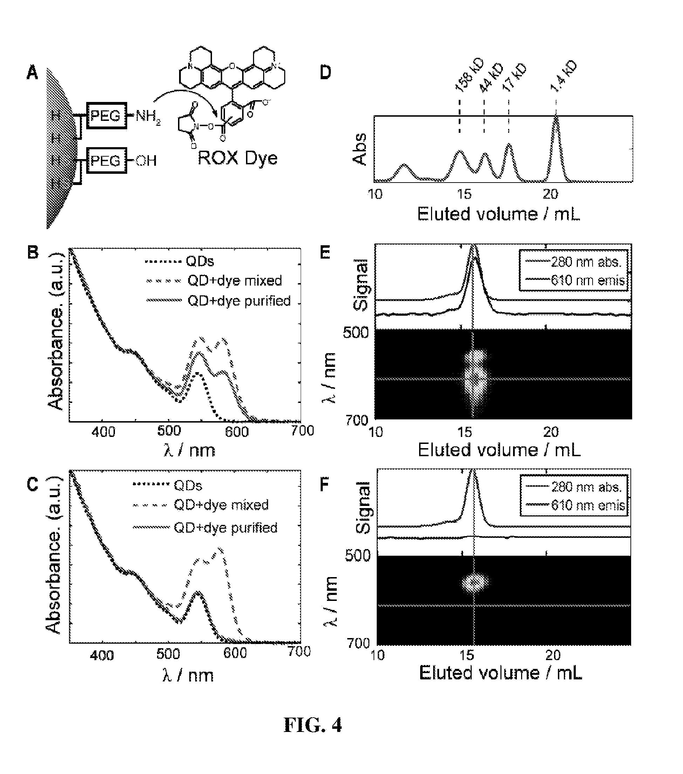

**Covalent conjugation to a dye for FRET-based sensing.** In order to evaluate the suitability of mixed amine/hydroxyl-PEG capped nanocrystals for routine derivatization, nanocrystals coated with 80% -OH and 20% -NH₂ terminated ligands were reacted with the amine-reactive *N*-hydroxysuccinimidyl ester of carboxy-X-rhodamine (ROX), a red-emitting organic fluorescent dye (FIG. 4A). The spectral overlap between the nanocrystal photoluminescence and dye absorbance affords efficient Forster Resonance Energy Transfer (FRET) from the nanocrystal to the dye, and as such, this system serves as a good model for chemically sensitive nanocrystal-dye energy transfer systems that have recently been investigated (Snee, P. T.; et al., J. Am. Chem. Soc. 2006, 128, 13320-13321).

Following reaction with the ROX succinimidyl ester, the nanocrystals were separated from unbound dye and NHS byproduct via ultrafiltration. UV-Vis absorption spectra were used to monitor the dye:nanocrystal molar ratio before and after purification. FIG. 4B shows the absorption spectra of the starting nanocrystals, the reaction mixture, and the purified product, with the dye absorption peak clearly visible. A fit of the spectrum as a sum of nanocrystal and dye contributions revealed a dye:nanocrystal ratio of 5.0 as-mixed, and 2.9 upon purification, indicating a coupling yield of 58%. In a control experiment (FIG. 4C), a nanocrystal sample from the same batch was mixed with the free-acid form of ROX under identical reaction conditions. Upon purification, less than 3.4% of the dye remained, as expected since no reaction of primary amines with free carboxylic acids was expected under the mild conditions of the conjugation procedure.

The FRET efficiency of the nanocrystal-dye couple was estimated to be ~90% by measuring the amount of nanocrystal fluorescence quenching after dye conjugation. From the measured FRET efficiency, the measured amount of dye per nanocrystal, and the Förster distance (R₀ = 5.6 nm), the separation distance was calculated to be r = 4.8 nm (see, e.g. Aaron R. Clapp, et al. ChemPhysChem 2006, 7, 47-57). The purified nanocrystal-ROX conjugate was additionally characterized by gel filtration chromatography (GFC) with in-line detection of absorbance at 280 nm and acquisition of full spectrum fluorescence with excitation at 460 nm. Comparison with protein molecular weight standards (FIG. 4D) of known hydrodynamic diameter (HD) provided an indication of size for samples under investigation. FIG. 4E shows the GFC results for the purified nanocrystal-ROX conjugate shown in FIG. 4B. A single UV absorption feature was detected at 15.7 mL eluted volume, corresponding to a protein-equivalent molecular weight of -94 kD, or a HD of -8.2 nm, in reasonable agreement with the HD obtained by DLS. The emission spectrum at this volume showed two prominent features at 558 nm and 610 nm corresponding to the nanocrystal and ROX emission wavelengths, respectively. Presence of ROX emission at an elution volume corresponding to the nanocrystals indicates that the nanocrystal and dye are indeed bound.

By contrast, GFC of a mixture of free nanocrystals and ROX dye at the same dye:nanocrystal ratio as the coupled sample showed UV absorbance and nanocrystal emission signals at the same elution volume as the conjugated couple, but no dye fluorescence (FIG. 4F). Indeed, the dye was not detected at all because of its low absorption cross section at the absorbance and excitation wavelengths used by the instrument: ROX emission was detected in the coupled case because dye molecules bound to the nanocrystal are excited by FRET. Uncoupled nanocrystals also elute at 15.7 mL in this experiment, suggesting that attachment of a dye did not significantly alter the nanocrystal HD. Together, the GFC data indicated that small-molecule dyes in NHS ester form can be covalently bound to the mixed ligand nanocrystals without perturbing the nanocrystal size, and that control experiments with non-activated dye show no indication of coupling/binding.

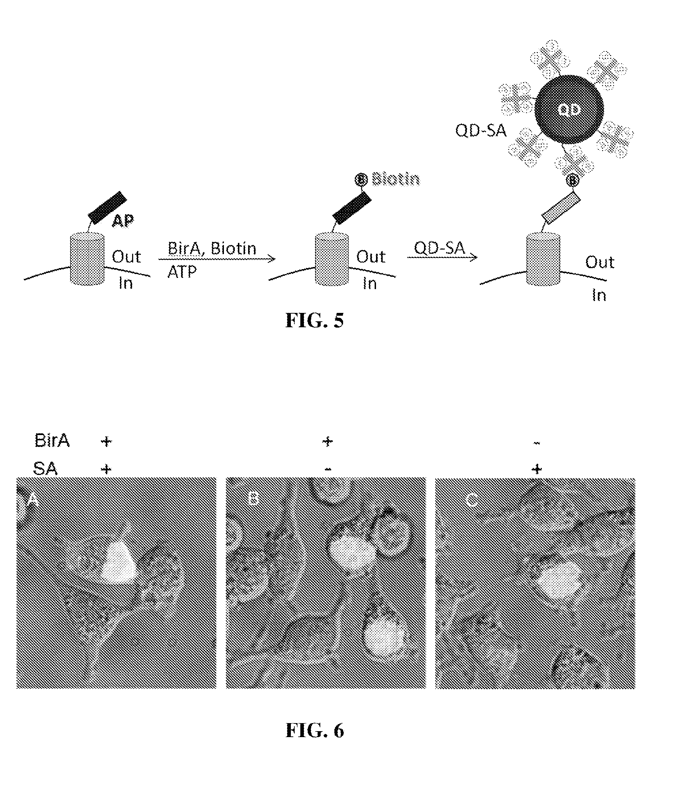

**Covalent conjugation to streptavidin for high affinity cell labeling.** A labeling construct that linked a nanocrystal directly to membrane proteins via a streptavidin/biotin interaction was designed This approach avoided the need for bulky intermediary primary and secondary antibodies (see, for example, Wu, X.; et al., Nature Biotechnol. 2003, 21, 41-46). The direct labeling was made possible by the development of biotin ligase (BirA) for the site-specific biotinylation of a 15 amino-acid recognition sequence called the acceptor peptide (AP) fused to either terminus of the receptor of interest (FIG. 5; Howarth, M.; et al., PNAS. 2005, 102, 7583-7588). The addition of recombinant BirA, biotin and ATP to the cell medium enables specific and efficient biotinylation of the AP tag, and nanocrystals conjugated to streptavidin (nanocrystal-SA) can then be applied to visualize the biotinylated protein population.

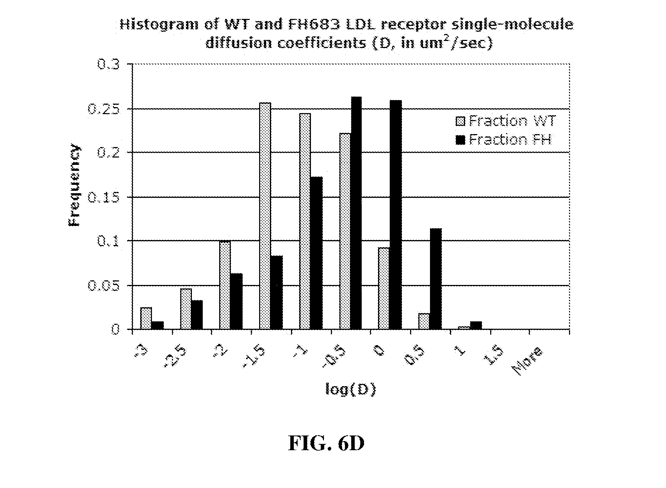

Accordingly, SA was conjugated to 20% -NH₂/80% -OH DHLA-PEG coated nanocrystals. SA was first activated using 1000 equivalents of EDC/NHS in MES buffer (pH 5.5) for 30 min. For coupling to proceed efficiently, it was important to remove excess coupling reagent by ultrafiltration before applying the activated SA to amine-functionalized nanocrystals. The activated SA was mixed with nanocrystals in bicarbonate buffer (pH 8.4), allowed to react for 1 hr, and purified by ultrafiltration. Nanocrystal-SA was applied to HeLa cells transfected with a low density lipoprotein receptor-acceptor peptide fusion construct (LDLR-AP), along with enhanced yellow fluorescent protein (EYFP) as a nuclear co-transfection marker. Thus, cells which display EYFP fluorescence from the nucleus also contain LDLR-AP on the cell surface. Nanocrystal-SA was applied at 50 nM to the cell medium after biotinylation of LDLR-AP and observed specific binding of the nanocrystal to the surface of transfected cells (FIG. 6A). Adjacent untransfected cells, indicated by the absence of the EYFP nuclear marker, did not show any nanocrystal-SA binding, illustrating the high specificity of labeling. Control experiments in which unconjugated nanocrystals were applied (FIG. 6B), and in which BirA was omitted (FIG. 6C) also showed no binding. In FIG. 6, red shows the NC605 channel; green, the EYFP channel. FIG. 6A is an image of AP-LDLR receptors on HeLa cells biotinylated with BirA, and labeled using streptavidin covalently conjugated to 20% -NH/-OH DHLA-PEG coated nanocrystals. FIGS. 6B and 6C are control experiments with unconjugated nanocrystals, and without BirA, resepectively.

Monovalent nanocrystals were used to study the mobility of a mutant of low density lipoprotein (LDL) receptor with a truncated cytosolic tail, originally found from an individual with Familial Hypercholesterolaemia (PH) (see, for example, Hobbs,H.H., et al., Annu. Rev. Genet. 24, 133-170 (1990)). The FH phenotype has been extensively analyzed by following the ligand of the receptor, LDL, but this method analyzed the behavior of the receptor itself. A pulse-chase with mSA-Alexa Fluor 568 to confirmed faster internalization of wt AP-tagged receptor compared to FH. Single monovalent nanocrystals bound to the biotinylated AP-LDL receptor were imaged, as indicated by the nanocrystal fluorescence intensity and blinking. The mobility of receptors labeled with monovalent nanocrystals was significantly greater for FH than wild-type LDL receptor (p=1.6x10⁻¹⁴) (Fig. 6D), consistent with tethering of the wild-type cytosolic tail by adaptors in clathrin-coated pits (see, e.g., Michaely,P., et al., J. Biol. Chem. 279, 34023-34031 (2004)).

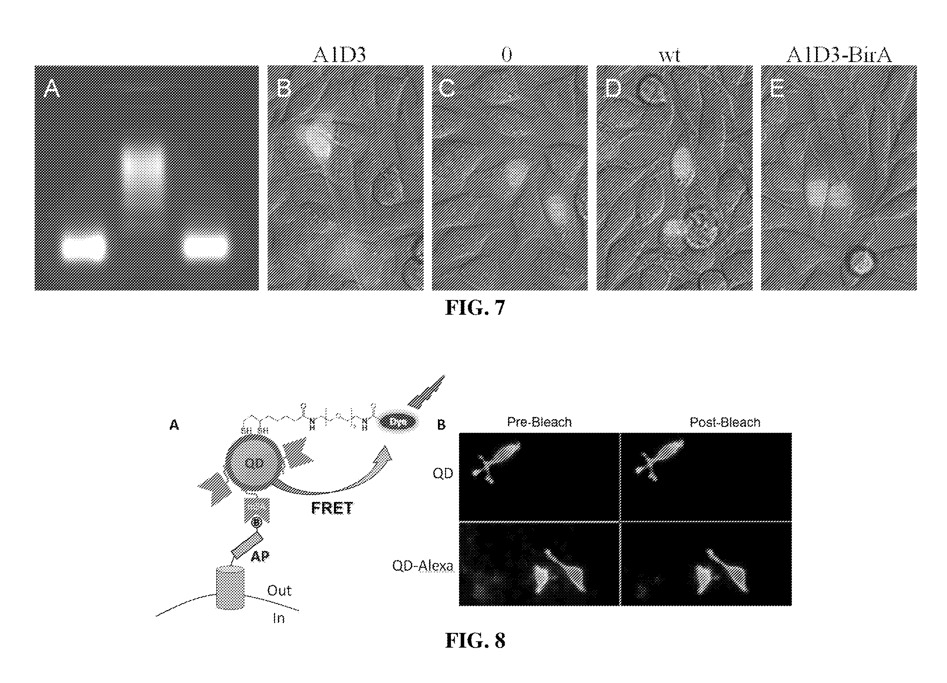

FIG. 6D presents results of single molecule tracking of the LDL receptor with monovalent nanocrystals. Histograms show distinct distributions of single-molecule diffusion coefficients for wild-type and FH LDL receptor (Kolmogorov-Smirnov D' statistic = 0.317, one sided p=1.6x10⁻¹⁴). Mean log(D) for WT, -1.36 (416 tracks). For FH, -0.84 (256 tracks). **His₆-tag conjugation of proteins.** DHLA-PEG-CO₂H coated nanocrystals exhibited high mobility on agarose gel. We incubated these nanocrystals with a His₆-tagged mono-valent variant of streptavidin (mSA) containing a single biotin binding site (see Howarth, M.; et al., Nat Meth 2006, 3, 267-273), and observed a significant decrease in the gel mobility of the nanocrystal (FIG. 7A), most likely due to the metal-affinity driven self-assembly of the protein onto the nanocrystal surface (nanocrystal-mSA). See, e.g., Pons, T.; et al., J. Phys. Chem. B 2006, 110, 20308-20316,

Incubation with wild-type streptavidin (wtSA), which does not feature a His₆-tag, resulted in no change in nanocrystal mobility on the gel (FIG. 7A). To show that the conjugated protein retained biological functionality, nanocrystal-mSA was tested in cell labeling. Addition of nanocrystal-mSA to HeLa cells displaying biotinylated AP-LDLR resulted in a high degree of specific binding (FIG. 7B). Control experiments where labeling was attempted using unconjugated nanocrystals (FIG. 7C), nanocrystals incubated with wtSA (FIG. 7D), and nanocrystal-mSA with without addition of BirA (FIG. 7E) all resulted in the lack of binding, as was expected. Furthermore, the stability, specificity, and high QY of these nanocrystals enabled single particle tracking of LDLR on the cell surface over extended periods.

Conjugation to mSA was achieved simply by mixing the desired ratio of nanocrystals to His₆-tagged protein followed by incubating at RT for 1 hr. No coupling agents, or purification steps were required to produce nanocrystals suitable for cell labeling, highlighting the ease of this conjugation method in producing biologically functional nanocrystals for targeted cell imaging applications. Furthermore, a unique property of the nanocrystal gel shift after His₆-tag protein conjugation is that a given ratio of mSA to nanocrystals will produce a ladder of bands following a Poisson intensity distribution, with each band representing nanocrystals bound to discrete numbers of mSA (see, e.g., Pons, T.; Uyeda, H. T.; Medintz, I. L.; Mattoussi, H., J. Phys. Chem. B 2006, 110, 20308-20316). If the first band can be isolated, corresponding to nanocrystals conjugated to exactly one mSA, then a truly mono-valent nanocrystal can be prepared. The single biotin binding site of mSA, along with a single copy of mSA per nanocrystal reduces the amount of binding sites per nanocrystal to exactly one, eliminating any possibility of cross-linking protein targets, a major issue with commercially available nanocrystals that contain up to 20 wtSA bound per nanocrystal, and thus up to 80 binding sites per nanocrystal. See, for example, Medintz, I.; et al, Nature Mater. 2005, 4, 435-446.

**Applying covalent and non-covalent conjugation strategies.** NC565 coated with 20% -NH₂/80% -OH DHLA-PEG was first conjugated to Alexa 568 NHS-ester via amide bond formation. After purification by ultrafiltration, the average number of dye to nanocrystal was calculated to be ~1.3 from the UV-vis absorbance spectrum. Excitation at 420 nm, where there is minimal dye absorbance, resulted in both nanocrystal and dye emission, suggesting the occurrence of FRET. Based on the quenching of the nanocrystal fluorescence compared to nanocrystals without dye bound, the FRET efficiency was estimated to be ~74%. The nanocrystal-dye conjugate was then incubated with mSA and applied to HeLa cells displaying biotinylated AP-LDLR for labeling (FIGS. 8A-8B). Dual emission from nanocrystal and dye were visible in the green and red channels, respectively, and co-localization of the two channels on the targeted cells verified the integrity of the nanocrystal-dye-mSA conjugate (FIG. 8B). Upon intense irradiation at 420 nm excitation for an extended period, bleaching of the dye was observed in the red channel, along with a recovery of the nanocrystal emission in the green channel, further suggesting the occurrence of FRET from the targeted nanocrystal-dye on the cell surface. A control sample using nanocrystal-mSA without dye bound showed no difference in the ratio of the red and green channels after extended irradiation.

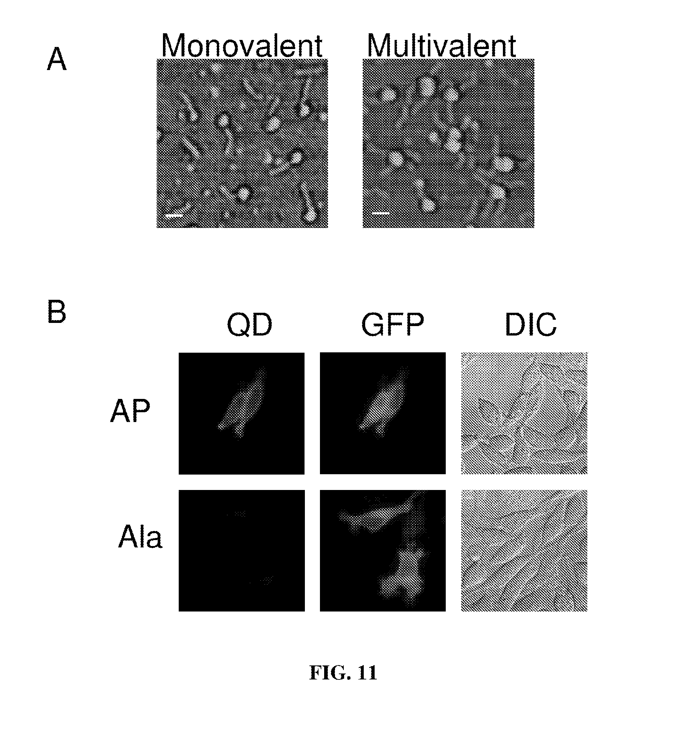

**Characterization of monovalent nanocrystals.** To confirm the valency of the isolated nanocrystals, we performed atomic force microscopy (AFM) on monovalent nanocrystals incubated with a 3-fold excess of mono-biotinylated DNA (FIG. 11A). AFM allowed single molecules of nanocrystal and DNA to be visualized and showed nanocrystals bound to a single biotinylated DNA, supporting the presence of a single biotin binding site per nanocrystal. When nanocrystals conjugated with several copies of monovalent streptavidin (multivalent nanocrystals) were similarly analyzed, multiple copies of DNA attached to the nanocrystal. Multivalent nanocrystals appeared larger by AFM as a result of the contribution of the extra copies of mSA to nanoparticle size.

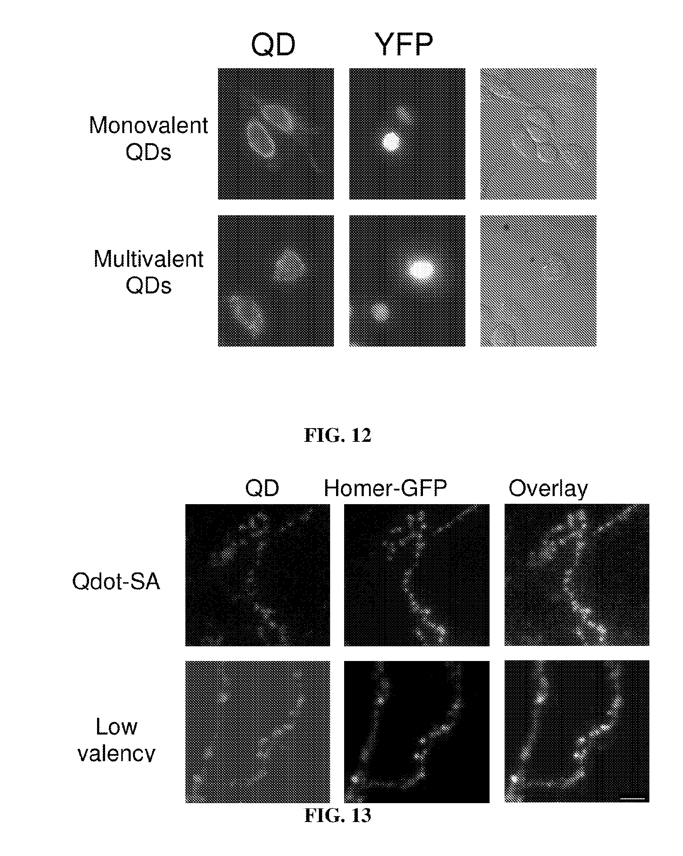

Nanocrystals bound to a single copy of a monovalent antibody fragment were also purified by agarose electrophoresis. See FIG. 10B. A single-chain Fv antibody which had been selected by yeast surface display to bind exceptionally tightly to the tumor marker carcinoembryonic antigen was chosen (Graff *et al*., 2004). Monovalent antibody-nanocrystals were purified in the same way (FIGS. 13A-13B) and specifically labeled carcinoembyonic antigen expressed at the cell surface (FIG. 13C). Monovalent antibody-nanocrystals did not label cells transfected instead with LDLR-AP and unconjugated nanocrystals did not bind carcinoembyonic antigen. Proteins needed to be >50 kDa for separation of nanocrystals conjugates according to valency. Electrophoretic separation of nanocrystals according to valency was also efficient on commercial polyacrylic acid-coated nanocrystals, but these nanocrystals gave unacceptably high non-specific sticking to cells. PEG-amine-coated nanocrystals had low non-specific binding but did not migrate sufficiently on the gel to allow separation according to valency, as previously observed. See, for example, So, M.K., et al. (2006). Nat.Biotechnol. 24, 339-343.

### Transfected BirA-ER allows targeting of monovalent nanocrystals.

Acceptor-peptide tagged proteins on the surface of living cells can be biotinylated by adding recombinant biotin ligase to the medium (Howarth *et al*., 2005;Chen *et al*., 2005). To simplify the labeling of receptors with streptavidin-nanocrystals for cell biology and potential applications in living animals, cells were transected with a biotin ligase that was targeted to the endoplasmic reticulum (ER) with an immunoglobulin signal sequence and retained in the endoplasmic reticulum by a KDEL C-terminal sequence (BirA-ER). Biotin ligase expressed in the secretory pathway has previously been used for gene therapy and antibody modification applications but not for receptor tracking. See, e.g., Nesbeth, D., et al. (2006). Mol. Ther. 13, 814-822; and Barat,B., Wu,A.M. (2007). Metabolic biotinylation of recombinant antibody by biotin ligase retained in the endoplasmic reticulum. Biomol.Eng.

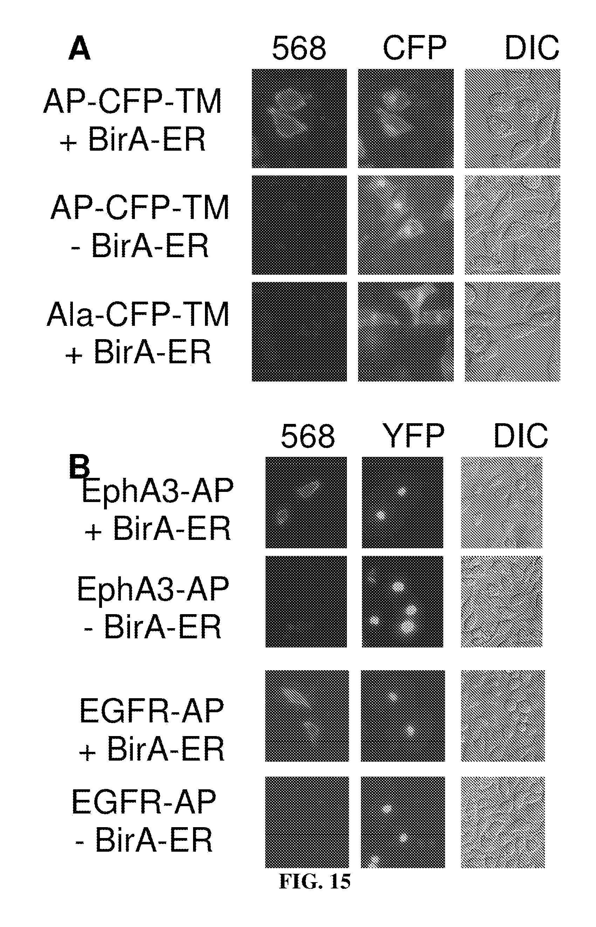

BirA-ER efficiently biotinylated AP fused to the low density lipoprotein receptor (LDLR), allowing cell surface labeling with streptavidin-dye (FIGS. 9B and 11B). BirA-ER did not biotinylate endogenous cell surface proteins, since cells are not stained with streptavidin if BirA-ER was expressed along with a negative control construct with a mutation in AP (LDLR-Ala) (FIG. 11B). This method was effective generally: BirA-ER was also used to label the Epidermal Growth Factor Receptor and EphA3 fused to AP in HeLa cells and GluR2-AP in neurons (FIG. 15B). This generality contrasts to a previous report of receptor biotinylation by cytosolic biotin ligase, which only biotinylated a subset of biotin acceptor domains. Biotin in normal growth medium (~9 nM) can be sufficient for biotinylation of AP fusions in the ER (see, e.g., Baumgartner, M.R., et al. (2004). Am.J.Hum.Genet. 75, 790-800), however, supplementing the growth medium with biotin on the day of transfection can improve biotinylation efficiency. 10 µM biotin gave optimal biotinylation of LDLR-AP. Biotin even at 1 mM did not cause any apparent change in viability of HeLa or neuronal cultures. For neurons it was not necessary to supplement the medium with biotin to obtain efficient AP biotinylation by BirA-ER (FIG. 13).

Monovalent nanocrystals were used to label LDLR-AP biotinylated by BirA in the endoplasmic reticulum (FIG. 11B). In this case we used a version of BirA-ER containing yellow fluorescent protein (YFP), BirA-YFP-ER, which behaved equivalently to BirA-ER but also allowed visualization of transfected cells. Monovalent nanocrystals only bound to AP-expressing cells and did not bind to negative control cells expressing LDLR with a point mutation in the AP tag (LDLR-Ala). This indicated that monovalent nanocrystals retained similar high specificity of cellular labeling to commercial streptavidin-nanocrystals (Howarth *et al*., 2005).

**Nanocrystal monovalency avoids receptor activation and impairment of mobility.**

Receptor clustering is a common way to activate signal transduction. On the other hand, the nanoparticles used for single particle tracking are most often multivalent (see, for example, Saxton,M.J., Jacobson,K. (1997). Annu.Rev.Biophys.Biomol.Struct. 26, 373-399; and Jaiswal, J. K. and Simon, S. M. Trends Cell Biol. 14[9], 497-504.: 2004). The tyrosine kinase EphA3, a receptor for ephrin that has important roles in cell movement in development and metastasis, became phosphorylated when clustered by ephrin-coated beads. See, e.g., Wimmer-Kleika.mp,S.H., Lackmann,M. (2005). IUBMB.Life 57, 421-431; and Wimmer-Kleikamp,S.H., et al. (2004). J.Cell Biol. 164, 661-666, Controlled valence nanocrystals were used for imaging CHO cells expressing EphA3-AP. EphA3-AP was biotinylated and incubated at 37 °C with DHLA-PEG8-CO₂H nanocrystals bound to one or to multiple copies of monovalent streptavidin (FIG. 12). Monovalent nanocrystals labeled the receptor and remained diffusely localized on the cell surface. This pattern of staining was the same as when EphA3-AP was labeled with monovalent streptavidin conjugated to Alexa Fluor 568 dye. Multivalent nanocrystals, in contrast, clustered EphA3-AP and triggered internalization, just as was been observed with ephrin bead stimulation. Commercial (multivalent) streptavidin-nanocrystals similarly caused receptor clustering and internalization.

Receptor cross-linking can also cause a reduction in receptor mobility on the cell surface. The mobility of a mutant of low density lipoprotein receptor (LDLR) with a truncated cytosolic tail, originally found from an individual with Familial Hypercholesterolemia (FH), was examined. LDLR is the key receptor for uptake of cholesterol, in the form of low density lipoprotein, by the periphery. This truncated mutant abolishes retention of the receptor in coated pits and reduced endocytosis. LDLR has only previously been imaged at the single molecule level by labeling of its ligand, LDL. It is not yet clear if ligand-bound and -unbound receptor have the same trafficking behavior. The receptor, FH LDLR-AP, was labeled by biotinylating with exogenous biotin ligase and adding monovalent or multivalent nanocrystals. Single receptor bound nanocrystals could be imaged individually, as indicated by the nanocrystal fluorescent intensity and blinking (see, e.g., Nirmal,M., et al. (1996). Nature 383, 802-804). The mobility of receptors labeled with monovalent nanocrystal was substantially greater than those labeled with multivalent nanocrystals, consistent with receptor cross-linking by multivalent nanocrystals.

**Monovalent nanocrystals have a size comparable to an antibody.** For many cellular locations, the size of the nanoparticle attached to the receptor does not significantly affect receptor mobility. See, e.g., Borgdorff, A.J., Choquet,D. (2002) Nature 417, 649-653; Kusumi, A., et al. (2005). Annu.Rev.Biophys.Biomol.Struct. 34, 351-378; and Thoumine, O., et al. (2005) Biophys. J. 89, L40-L42. The transmembrane helices and interactions of the cytosolic tail are most often the determining factors for receptor mobility (Saxton and Jacobson, 1997). However, for certain confined locations, the nanoparticle size can significantly slow mobility or perturb labeling, notably neuronal synapses which are only 24-30 nm wide. See, e.g., Groc, L., et al. (2004). Nat.Neurosci. 7, 695-696; Howarth, M., et al. (2005). Proc.Natl.Acad.Sci.U.S.A. 102, 7583-7588; and Zuber, B., et al. (2005). Proc.Natl.Acad.Sci. U.S.A. 102, 9192-19197.

DHLA-PEG8-CO₂H nanocrystals emitting at 605 nm gave a hydrodynamic diameter of 11 nm, as determined by dynamic light scattering (DLS, Table 1), which is comparable to an IgG antibody (9.7 nm), the standard probe for cell surface labeling, and to unconjugated R-phycoerythrin (11 nm), a probe used in single particle tracking but with limited photostability. Commonly used commercial streptavidin nanocrystals emitting as 605 nm have a diameter of 21 nm. Generating nanocrystals bound to a single monovalent streptavidin, rather than multiple copies, should contribute to keeping the size as small as possible. We found only a 1.2 nm increase in diameter upon the attachment of a single monovalent streptavidin to the nanocrystal. An alternative method of measuring nanocrystal size, fluorescence correlation spectroscopy (FCS), gave similar values (Table 1).

**Table 1**

| Nanocrystal sample | Hydrodynamic diameter by DLS (nm) | Hydrodynamic diameter by FCS (nm) |
|---|---|---|
| Unconjugated | 11.1 ± 0.1 | 10.1 ± 0.6 |
| Monovalent | 12.3 ± 0.2 | 13.4 ± 0.6 |
| Commercial nanocrystal/wild type streptavidin | 21.2 ± 0.2 | 23.4 ± 0.8 |

**Small nanocrystals improve synaptic staining of AMPA receptors.** Reducing the size of streptavidin-nanocrystals from 21 nm to 12 nm improved nanocrystal access to synapses for labeling the AMPA receptor subunit GluR2. Hippocampal neurons in dissociated culture were cotransfected with GluR2-AP, the synaptic marker Homerlb-GFP (see Xiao,B., et al. (1998). Neuron 21, 707-716) and BirA-ER. GluR2-AP was biotinylated by BirA-ER and the surface was labeled with monovalent nanocrystals or commercial streptavidin nanocrystals. The colocalization of nanocrystals and synapses was then compared (FIG. 13). As previously observed (Howarth *et al*., 2005), commercial nanocrystals were to a large extent excluded from synapses. Monovalent nanocrystal labeling, however, was concentrated at synapses, consistent with the reduced size of these nanocrystals and improving the ability to faithfully label GluR2 (see, for example, Passafaro,M., et al. (2001). Nat.Neurosci. 4, 917-926).

**EGFR Tracking.** The aminoNC-SA conjugates were applied to specific cell labeling and single particle tracking of EGF receptors (EGFR) on live cells. EGFR is an important activator of cell division and a target for therapy of many cancers (see, e.g., Moasser, M. M., Oncogene 2007, 26, 6577-92). There are still many questions about the mechanism of receptor association and internalization in EGFR signal transduction, which are best addressed through study at the single molecule level. See, for example, Lidke, D. S.; et al, J. Cell. Biol. 2005, 170, 619-626, and Teramura, Y.; et al., EMBO J. 2006, 25, 4215-22.

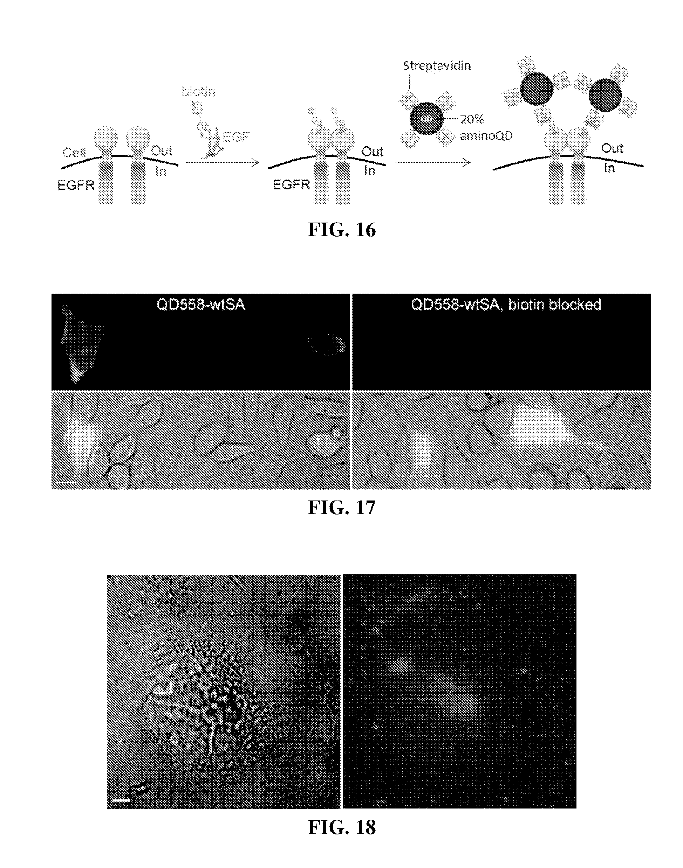

Figure 16 schematically illustrates the targeting of live cells transfected with human EGFR. COS7 cells were incubated with biotinylated EGF (bioEGF) and then stained with aminoNC-SA. Specific binding of the nanocrystals to the surface of EGFR transfected cells was observed, shown by the blue fluorescent protein (BFP) co-transfection marker. See the left panel of Figure 17, which shows targeting of 20% aminoNC-SA conjugates to EGFR on live cells. EGFR expressing cells are indicated by BFP co-transfection marker. Top row: NC558 channel. Bottom row: BFP + DIC channel. Left: EGFR transfected COS7 cells treated with biotinylated EGF and stained with aminoNC-SA. Right: control experiment in which aminoNC-SA was blocked with excess free biotin. Scale bar, 10 µm. Adjacent untransfected cells, indicated by the absence of the BFP marker, did not show nanocrystal staining, illustrating the specificity of labeling.

Furthermore, the photostability, specificity, and high QY of these nanocrystals enabled single particle tracking of EGF interaction with EGFR on the cell surface (Figure 18 Left: DIC channel. Right: NC605 channel. Large patches of brightness represent autofluorescence, bright dots represent clusters of nanocrystals, scale bar, 5 µm). Individual nanocrystals were identified by their fluorescence intensity and intermittency, or blinking, behavior and could be seen in motion on the surface of cells in a manner consistent with active transport of the labeled receptor.

Other embodiments are within the scope of the following claims.

## Claims

1. A method of making a controlled valency semiconductor nanocrystal, comprising:
contacting a population of semiconductor nanocrystals with a compound having an affinity for the semiconductor nanocrystal to form a distribution of compound-associated nanocrystals; and
separating the members of the distribution of compound-associated nanocrystals according to the number of compounds associated with each nanocrystal; and
isolating members of the distribution of compound-associated nanocrystals which have exactly one compound associated with a nanocrystal, wherein the isolated semiconductor nanocrystal is monovalent and is associated with exactly one binding site for an affinity tag or affinity target;
wherein the compound is a monovalent avidin or a monovalent streptavidin.

2. The method of claim 1, wherein the compound includes a polyhistidine tag.

3. A method of imaging a single particle, comprising:
joining an affinity tag to a cell-surface protein;
contacting the cell with a composition comprising a valency controlled semiconductor nanocrystal associated with exactly one compound having exactly one binding site for the affinity tag, wherein the semiconductor nanocrystal is associated with exactly one monovalent avidin or exactly one monovalent streptavidin; and
imaging the cell and semiconductor nanocrystal substantially simultaneously.

4. The method of claim 3, wherein the affinity tag is biotin.

5. The method of claim 4, wherein joining an affinity tag to a cell-surface protein includes contacting a fusion protein including an acceptor peptide (AP) sequence with a biotin ligase.

## Patentansprüche

1. Verfahren zur Herstellung eines Halbleiter-Nano-kristalls mit kontrollierter Valenz, umfassend:
Inkontaktbringen einer Population von Halbleiter-Nanokristallen mit einer Verbindung mit einer Affinität für den Halbleiter-Nanokristall unter Bildung einer Verteilung von verbindungsassoziier-ten Nanokristallen; und
Trennen der Mitglieder der Verteilung von verbindungsassoziierten Nanokristallen gemäß der Anzahl an mit jedem Nanokristall assoziierten Verbindungen; und
Isolieren von Mitgliedern der Verteilung von verbindungsassoziierten Nanokristallen, bei denen genau eine Verbindung mit einem Nanokristall assoziiert ist, wobei der isolierte Halbleiter-Nanokristall monovalent ist und mit genau einer Bindungsstelle für ein Affinitäts-Tag oder Affinitätsziel assoziiert ist;
wobei es sich bei der Verbindung um ein monovalentes Avidin oder ein monovalentes Streptavidin handelt.

2. Verfahren nach Anspruch 1, wobei die Verbindung ein Polyhistidin-Tag enthält.

3. Bildgebungsverfahren für ein einzelnes Partikel, umfassend:
Verbinden eines Affinitäts-Tag mit einem Zelloberflächenprotein;
Inkontaktbringen der Zelle mit einer Zusammensetzung, die einen mit genau einer Verbindung mit genau einer Bindungsstelle für das Affinitäts-Tag assoziierten Halbleiter-Nanokristall mit kontrollierter Valenz umfasst, wobei der Halbleiter-Nanokristall mit genau einem monovalenten Avidin oder
genau einem monovalenten Streptavidin assoziiert ist; und
weitgehend gleichzeitige Bildgebung der Zelle und des Halbleiter-Nanokristalls.

4. Verfahren nach Anspruch 3, wobei es sich bei dem Affinitäts-Tag um Biotin handelt.

5. Verfahren nach Anspruch 4, wobei das Verbinden eines Affinitäts-Tag mit einem Zelloberflächenprotein das Inkontaktbringen eines eine Akzeptorpeptid(AP)-Sequenz enthaltenden Fusionsproteins mit einer Biotin-Ligase beinhaltet.

## Revendications

1. Procédé de fabrication d'un nanocristal semi-conducteur à valence contrôlée, comprenant :
la mise en contact d'une population de nanocristaux semi-conducteurs avec un composé ayant une affinité pour le nanocristal semi-conducteur pour former une distribution de nanocristaux associés à un composé ; et
la séparation des membres de la distribution de nanocristaux associés à un composé en fonction du nombre de composés associés à chaque nanocristal ; et
l'isolement des membres de la distribution de nanocristaux associés à un composé qui ont exactement un composé associé à un nanocristal, le nanocristal semi-conducteur isolé étant monovalent et étant associé à exactement un site de liaison pour un marqueur d'affinité ou une cible d'affinité ;
le composé étant une avidine monovalente ou une streptavidine monovalente.

2. Procédé selon la revendication 1, dans lequel le composé comporte un marqueur polyhistidine.

3. Procédé d'imagerie d'une particule individuelle, comprenant :
la liaison d'un marqueur d'affinité à une protéine de surface cellulaire ;
la mise en contact de la cellule avec une composition comprenant un nanocristal semi-conducteur à valence contrôlée associé à exactement un composé ayant exactement un site de liaison pour le marqueur d'affinité, le nanocristal semi-conducteur étant associé à exactement une avidine monovalente ou exactement une streptavidine monovalente ; et
l'imagerie de la cellule et du nanocristal semi-conducteur de façon sensiblement simultanée.

4. Procédé selon la revendication 3, dans lequel le marqueur d'affinité est la biotine.

5. Procédé selon la revendication 4, dans lequel la liaison d'un marqueur d'affinité à une protéine de surface cellulaire comporte la mise en contact d'une protéine de fusion comportant une séquence de peptide accepteur (AP) avec une biotine ligase.
